# FASCICULE DE BREVET EUROPEEN

(11) **EP 0 662 971 B1**
(45) Date de publication et mention de la délivrance du brevet: **13.05.1998**
(21) Numéro de dépôt: 93920940.9
(22) Date de dépôt: 28.09.1993
(51) Int. Cl.: C07D 487/04, A61K 31/495

(54) **DERIVES DE 5H,10H-IMIDAZO[1,2-a]INDENO[1,2-e]PYRAZINE-4-ONE, LEUR PREPARATION ET LES MEDICAMENTS LES CONTENANT**
5H,10H-IMIDAZO[1,2-a]INDENO[1,2-e]PYRAZIN-4-ONE DERIVATE, IHRE HERSTELLUNG UND DIE SIE ENTHALTENDEN ARZNEIMITTEL
5H,10H-IMIDAZO[1,2-a]INDENO[1,2-e]PYRAZINE-4-ONE DERIVATIVES, PREPARATION THEREOF AND MEDICAMENTS CONTAINING THEM

(30) Priorité: 02.10.1992 FR 9211674
(43) Date de publication de la demande: 19.07.1995
(73) Titulaire: RHONE-POULENC RORER S.A., 92160 Antony (FR)
(72) Inventeur: ALOUP, Jean-Claude, F-94290 Villeneuve-le-Roi (FR); AUDIAU, François, F-94220 Charenton-le-Pont (FR); DAMOUR, Dominique, F-75014 Paris (FR); GENEVOIS-BORELLA, Arielle, F-94600 Choisy-le-Roi (FR); JIMONET, Patrick, F-78450 Villepreux (FR); MIGNANI, Serge, F-92290 Chatenay-Malabry (FR)
(74) Mandataire: Savina, Jacques
(86) Numéro de dépôt international: FR9300946
(87) Numéro de publication internationale: WO9407893

(56) Documents cités:
- EP-A- 0 013 914
- EP-A- 0 040 401
- EP-A- 0 074 929
- EP-A- 0 368 652
- WO-A-91/13878
- WO-A-91/16325
- DATABASE WPI Week 9232, Derwent Publications Ltd., London, GB; AN 92-263039
- CHEMICAL ABSTRACTS, vol. 94, no. 19, 11 Mai 1981, Columbus, Ohio, US; abstract no. 156866p, E. ABIGNENTE ET AL.
- CHEMICAL ABSTRACTS, vol. 107, no. 15, 12 Octobre 1987, Columbus, Ohio, US; abstract no. 134286, D. D. DAVEY. cité dans la demande

## Description

La présente invention concerne les composés de formule : 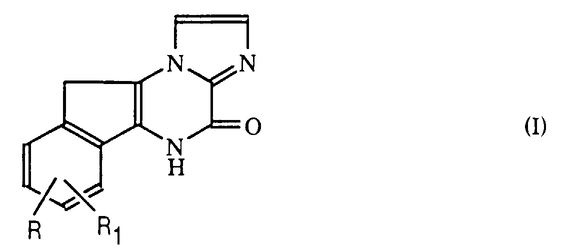 leurs sels, leur préparation, les intermédiaires pour les préparer et les médicaments les contenant.

Dans la formule (I), R et R₁, identiques ou différents, représentent un atome d'hydrogène ou d'halogène ou un radical alkyle, alcoxy, amino, acylamino, phényluréido, -N=CH-N(R₂)R₃, nitro, imidazolyle, phényle, SO₃H ou cyano, R₂ et R₃, identiques ou différents, représentent chacun un radical alkyle.

Sauf mention contraire, dans les définitions qui précédent et celles qui suivent, les radicaux alkyle et alcoxy contiennent 1 à 4 atomes de carbone en chaîne droite ou ramifiée et les radicaux et portions acyle contiennent 2 à 5 atomes de carbone. Les atomes d'halogène sont de préférence les atomes de brome, de chlore et de fluor.

Les composés de formule (I) pour lesquels R et/ou R₁ représentent un radical -N=CH-N(R₂)R₃ présentent des formes isomères E et Z. Ces isomères et leurs mélanges font partie de l'invention.

Les composés de formule (I) peuvent être préparés par désalkylation et désalification des dérivés de formule : 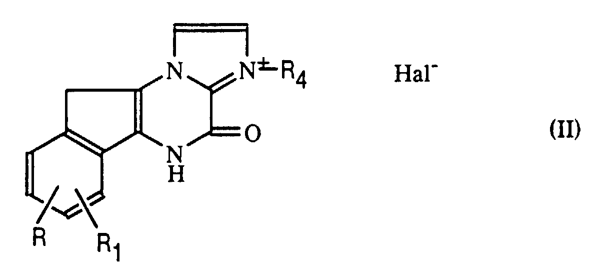 dans laquelle R et R₁ ont les mêmes significations que dans la formule (I), R₄ représente un radical alkyle et Hal représente un atome d'halogène et, de préférence, un atome de brome.

Cette réaction s'effectue, de préférence, en présence d'imidazole, à une température comprise entre 100 et 200°C et en particulier à 160°C.

Les dérivés de formule (II) sont nouveaux et font partie de l'invention.

Les dérivés de formule (II) peuvent être obtenus par action d'un dérivé de formule : 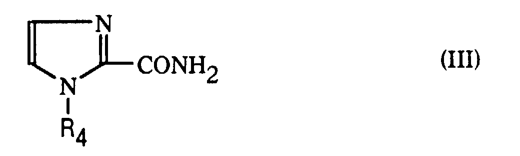 dans laquelle R₄ a les mêmes significations que dans la formule (II), sur une 2-halogénoindanone de formule : 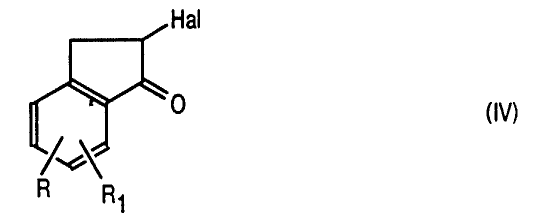 dans laquelle R et R₁ ont les mêmes significations que dans la formule (I) et Hal représente un atome d'halogène et, de préférence, un atome de brome.

Cette réaction s'effectue généralement au sein d'un solvant inerte tel que le diméthylformamide, à une température comprise entre 50 et 150°C et, de préférence à 115°C.

Les dérivés de formule (III) peuvent être obtenus par adaptation ou application de la méthode décrite par D. D. DAVEY, J. Org. Chem., 52, 4379 (1987).

Les dérivés de formule (IV) peuvent être obtenus par halogénation des indanones correspondantes au moyen d'un agent d'halogénation tel que le brome, le chlore, au sein d'un solvant inerte tel qu'un solvant chloré (chlorure de méthylène, chloroforme par exemple), à une température de -15°C ou au sein de l'acide acétique, à une température voisine de 20°C, ou un halogènure de cuivre, au sein du dioxanne, à une température voisine de 100°C ou par application ou adaptation des méthodes décrites par K. MORI, Agr. Biol. Chem., 27 (1), 22 (1963); J. CHAKRAVARTY, Indian J. Chem., 7 (3), 215 (1969), F. G. HOLLIMAN et coll., J. Chem. Soc., 9 (1960), D. MUKHOPADHYA et coll., J. Indian Chem. Soc., 47 (5), 450 (1970) et dans les brevets DE 2640358, EP 346107 et dans les exemples.

Les indanones peuvent être obtenues par application ou adaptation des méthodes décrites par M. OLIVIER et coll., Bull. Soc. Chim. de France, 3092 (1973), R. SEKA et coll., Chem. Ber., 75B, 1730 (1942), dans les brevets US 4263319, 4096173 et EP 314400 et dans les exemples.

Les composés de formule (I) peuvent également être préparés par cyclisation d'un dérivé de formule : 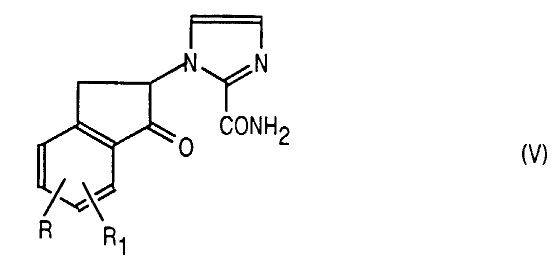 dans laquelle R et R₁ ont les mêmes significations que dans la formule (I).

Cette cyclisation s'effectue au moyen d'un acide tel que l'acide acétique, l'acide chlorhydrique, en milieu aqueux ou au sein d'un alcool tel que l'éthanol ou le méthanol, éventuellement en présence d'acétate d'ammonium, à la température d'ébullition du milieu réactionnel.

Les dérivés de formule (V) peuvent être obtenus par action d'ammoniac sur un dérivé de formule : 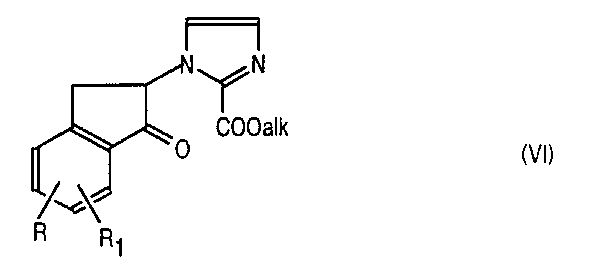 dans laquelle R et R₁ ont les mêmes significations que dans la formule (I) et alk représente un radical alkyle.

Cette réaction s'effectue au sein d'un alcool tel que le méthanol ou l'éthanol, à une température comprise entre 20°C et la température d'ébullition du milieu réactionnel.

Les dérivés de formule (VI) peuvent être obtenus par action d'une 2-halogénoindanone de formule (IV) sur un dérivé de formule : 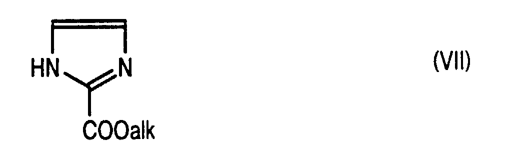 dans laquelle alk a les mêmes significations que dans la formule (VI).

Cette réaction s'effectue soit par fusion à une température comprise entre 130 et 180°C, soit au sein d'un solvant inerte tel que le diméthylformamide en présence d'une base telle qu'un hydrure de métal alcalin (hydrure de sodium par exemple), à une température voisine de 20°C, soit au sein d'un solvant inerte tel qu'un solvant chloré tel que le chloroforme en présence d'une base organique azotée (1,8-diazabicyclo[5,4,0] undéc-7-ène par exemple), à une température voisine de 20°C, soit au sein d'un solvant inerte tel qu'un alcool (éthanol, propanol par exemple), un solvant aromatique tel que le toluène, un solvant chloré (chloroforme par exemple), éventuellement en présence d'iodure de sodium, à la température d'ébullition du milieu réactionnel.

Les dérivés de formule (VII) peuvent être obtenus par application ou adaptation de la méthode décrite dans le brevet US 3600399.

Les composés de formule (I) pour lesquels R et/ou R₁ représentent un radical nitro peuvent également être préparés par nitration d'un composé de formule (I) correspondant pour lequel R et/ou R₁ représentent un atome d'hydrogène.

Cette nitration s'effectue généralement au moyen d'un agent de nitration tel qu'un nitrate de métal alcalin (nitrate de potassium de préférence), au sein de l'acide sulfurique, à une température de 0°C à 30°C.

Les composés de formule (I) pour lesquels R et/ou R₁ représentent un radical amino peuvent également être préparés par réduction d'un composé de formule (I) correspondant pour lequel R et/ou R₁ représentent un radical nitro.

Cette réduction s'effectue généralement au moyen d'hydrogène, sous une pression de 1 à 2 bar, en présence de soude aqueuse et d'un catalyseur tel que le palladium sur charbon, à une température voisine de 20°C.

Les composés de formule (I) pour lesquels R et/ou R₁ représentent un radical SO₃H peuvent également être préparés par sulfonation d'un dérivé de formule (I) correspondant pour lequel R et/ou R₁ représentent un atome d'hydrogène.

Cette réaction s'effectue généralement au moyen de l'acide chlorosulfonique, à une température comprise entre 0 et 20°C.

Les composés de formule (I) pour lesquels R et/ou R₁ représentent un radical acylamino peuvent également être préparés par acylation d'un composé de formule (I) correspondant pour lequel R et/ou R₁ représentent un radical amino.

Cette acylation s'effectue généralement au sein d'un solvant inerte tel que le diméthylformamide, au moyen d'un anhydride d'acide (alk-CO)₂O dans lequel alk représente un radical alkyle contenant 1 à 4 atomes de carbone, en présence d'une base telle qu'une trialkylamine (triéthylamine par exemple), à la température d'ébullition du milieu réactionnel.

Les composés de formule (I) pour lesquels R et/ou R₁ représentent un radical phényluréido peuvent également être préparés par action de l'isocyanate de phényle sur un composé de formule (I) correspondant pour lequel R et/ou R₁ représentent un radical amino.

Cette réaction s'effectue généralement au sein d'un solvant inerte tel que le diméthylsulfoxyde, le diméthylformamide, un solvant chloré (chloroforme par exemple), en présence d'une base telle qu'une trialkylamine (triéthylamine par exemple), à une température voisine de 20°C.

Les composés de formule (I) pour lesquels R et/ou R₁ représentent un radical -N=CH-N(R₂)R₃ peuvent également être préparés par action d'un composé de formule (I) correspondant pour lequel R et/ou R₁ représentent un radical amino sur un amide HCO-N(R₂)R₃ dans lequel R₂ et R₃ ont les mêmes significations que dans la formule (I).

Cette réaction s'effectue, de préférence, en présence de chlorure de méthylsulfonyle et d'une base telle qu'une trialkylamine (triéthylamine par exemple), à une température voisine de 20°C.

Les composés de formule (I) peuvent être purifiés par les méthodes connues habituelles, par exemple par cristallisation, chromatographie ou extraction.

Les isomères E et Z des composés de formule (I) pour lesquels R et/ou R₁ représentent un radical -N=CH-N(R₂)R₃ peuvent être séparés par les méthodes connues habituelles, par exemple par cristallisation ou chromatographie.

Les composés de formule (I) comportant un reste basique peuvent être éventuellement transformés en sels d'addition avec un acide minéral ou organique par action d'un tel acide au sein d'un solvant organique tel qu'un alcool, une cétone, un éther ou un solvant chloré.

Les composés de formule (I) comportant un reste acide peuvent éventuellement être transformés en sels métalliques ou en sels d'addition avec les bases azotées selon des méthodes connues en soi. Ces sels peuvent être obtenus par action d'une base métallique (alcaline ou alcalinoterreuse par exemple), de l'ammoniac, d'une amine ou d'un sel d'une amine sur un composé de formule (I), dans un solvant. Le sel formé est séparé par les méthodes habituelles.

Ces sels font également partie de l'invention.

Comme exemples de sels pharmaceutiquement acceptables, peuvent être cités les sels d'addition avec les acides minéraux ou organiques (tels que acétate, propionate, succinate, benzoate, fumarate, maléate, oxalate, méthanesulfonate, iséthionate, théophyllinacétate, salicylate, méthylène-bis-β-oxynaphtoate, chlorhydrate, sulfate, nitrate et phosphate), les sels avec les métaux alcalins (sodium, potassium, lithium) ou avec les métaux alcalinoterreux (calcium, magnésium), le sel d'ammonium, les sels de bases azotées (éthanolamine, triméthylamine, méthylamine, benzylamine, N-benzyl-β-phénéthylamine, choline, arginine, leucine, lysine, N-méthyl glucamine).

Les composés de formule (I) présentent des propriétés pharmacologiques intéressantes. Ces composés sont des antagonistes du récepteur de l'acide α-amino-3-hydroxy-5-méthyl-4-isoxazolepropionique (AMPA), connu aussi sous le nom de récepteur du quisqualate

Par ailleurs, les composés de formule (I) sont des antagonistes non compétitifs du récepteur N-méthyl-D-aspartate (NMDA) et, plus particulièrement, ce sont des ligands pour les sites modulateurs de la glycine du récepteur NMDA.

Ces composés sont donc utiles pour traiter ou prévenir toutes les ischémies (telles l'ischémie focale ou globale) consécutives à des accidents vasculaires cérébraux, un arrêt cardiaque, une hypotension artérielle, une intervention chirurgicale cardiaque ou pulmonaire ou une hypoglycémie sévère. Ils sont également utiles dans le traitement des effets dus à une anoxie, qu'elle soit périnatale ou consécutive à une noyade ou à des lésions cérébro-spinales. Ces composés peuvent également être utilisés pour traiter ou prévenir l'évolution de maladies neurodégénératives, de la chorée d'HUNTINGTON, de la maladie d'ALZHEIMER, de la sclérose latérale amyotrophique, de l'atrophie olivo-pontocérébelleuse et de la maladie de PARKINSON. Ces composés peuvent aussi être utilisés vis-à-vis des manifestations épileptogènes et/ou convulsives, pour le traitement des traumatismes cérébraux et spinaux, pour le traitement de l'anxiété (KEHNE et coll., Eur. J. Pharmacol., 193, 283 (1991), de la dépression (TRULLAS et coll.,Eur. J. Pharmacol., 185, 1 (1990), de la schizophrénie (REYNOLDS, TIPS, 13, 116 (1992), en tant qu'analgésiques (DICKENSON et coll., Neurosc. Letters, 121, 263 (1991), antianorexiques (SORRELS et coll., Brain Res., 572, 265 (1992), antimigraineux, antiémétiques et pour traiter les empoisonnements par des neurotoxines ou d'autres substances agonistes du récepteur NMDA, ainsi que les troubles neurologiques associés aux maladies virales telles que le sida (LIPTON et coll., Neuron, 7, 111 (1991)), la rage, la rougeole et le tétanos (BAGETTA et coll., Br. J. Pharmacol., 101, 776 (1990). Ces composés sont aussi utiles pour la prévention des symptômes d'abstinence aux drogues et à l'alcool et de l'inhibition de l'accoutumance et de la dépendance aux opiacés.

L'affinité des composés de formule (I) vis-à-vis du récepteur AMPA a été déterminée en étudiant l'antagonisme de la fixation spécifique du [³H]-AMPA sur des membranes de cortex cérébral de rat (HONORE et coll., Neuroscience letters, 54, 27 (1985)). Le [³H]-AMPA est mis à incuber en présence de 0,2 mg de protéines à 4°C pendant 30 minutes dans du tampon KH₂PO₄ 10mM, KSCN 100mM, pH7,5. La fixation non spécifique est déterminée en présence de L-glutamate 1mM. La radioactivité liée est séparée par filtration sur filtres PHARMACIA (Printed Filtermate A). L'activité inhibitrice de ces produits est généralement inférieure à 100 µM.

L'affinité des composés de formule (I) pour le site glycine lié au récepteur NMDA a été déterminée en étudiant l'antagonisme de la fixation spécifique du [³H]-DCKA sur des membranes de cortex cérébral de rat selon la méthode décrite par T. CANTON et coll., J. Pharm. Pharmacol., 44, 812 (1992). Le [³H]-DCKA (20nM) est mis à incuber en présence de 0,1 mg de protéines à 4°C pendant 30 minutes dans du tampon HEPES 50 mM, pH7,5. La fixation non spécifique est déterminée en présence de glycine 1mM. La radioactivité liée est séparée par filtration sur filtres Whatman GF/B. L'activité inhibitrice de ces produits est généralement inférieure à 100 µM.

Les composés de formule (I) présentent une toxicité faible. Leur DL50 est supérieure à 50 mg/kg par voie IP chez la souris.

Les composés de formule (I) préférés sont ceux pour lesquels soit R représente un atome d'hydrogène et R₁ est en position -7, -8 ou -9 et représente un atome d'hydrogène ou d'halogène ou un radical alkyle, alcoxy, amino, acylamino, phényluréido, -N=CH-N(R₂)R₃, nitro, imidazolyle, phényle ou SO₃H; soit R représente un atome d'halogène et R₁ représente un atome d'halogène ou un radical nitro.

### EXEMPLE 1

1,8 g de 1-[2-(5-fluoro-1-oxo-indanyl)]imidazole-2-carboxamide est dissous dans 90 ml de méthanol bouillant et la solution additionnée de 0,1 g de noir décolorant est filtrée. Le filtre est lavé avec 20 ml de méthanol bouillant puis le filtrat et le lavage sont réunis, additionnés de 27 ml de solution aqueuse d'acide chlorhydrique 12N et conservés pendant 3 heures à 5°C. Les cristaux sont séparés par filtration, lavés 2 fois par 20 ml au total de méthanol glacé et séchés sous pression réduite (1 mm Hg; 0,13 kPa) à 60°C. On obtient ainsi 1 g de chlorhydrate de 8-fluoro-5H,10H-imidazo[1,2-a]indéno[1,2-e] pyrazine-4-one se décomposant sans fondre au-dessus de 300°C [Spectre de R.M.N. : (200 MHz; DMSO d6; δ en ppm) : 4,11 (s, 2H : -CH2- en 10); 7,32 (ddd, J=9,5 - 8,5 et 2 Hz, 1H : -H7); 7,57 (dd, J=9,5 et 2 Hz, 1H : -H9); 7,97 (dd, J=8,5 et 5 Hz, 1H : -H6); 7.99 et 8,28 (2d, J=1,5 Hz, 1H chacun : -H de l'imidazole); 13,13 (mf, 1H : -CONH-)].

Le 1-[2-(5-fluoro-1-oxo-indanyl)]imidazole-2-carboxamide peut être préparé de la façon suivante : 2,2 g de 1-[2-(5-fluoro-1-oxo-indanyl)]imidazole-2-carboxylate d'éthyle sont dissous dans 80 ml d'une solution 2,5 N de méthanol ammoniacal et la solution est conservée pendant 20 heures à une température voisine de 20°C puis concentrée à sec sous pression réduite (15 mm Hg; 2 kPa) à 35°C. Le produit obtenu est mis en suspension dans 50 ml d'oxyde d'isopropyle, filtré, lavé 2 fois par 20 ml au total d'oxyde d'isopropyle puis séché sous pression réduite (15 mm Hg; 2 kPa) à une température voisine de 20°C. On obtient ainsi 1,85 g de 1-[2-(5-fluoro-1-oxo-indanyl)]imidazole-2-carboxamide sous forme d'un solide fondant à 221°C.

Le 1-[2-(5-fluoro-1-oxo-indanyl)]imidazole-2-carboxylate d'éthyle peut être préparé de la façon suivante : une solution de 13,3 g d'imidazole-2-carboxylate d'éthyle dans 145 ml de diméthylformamide anhydre est additionnée goutte à goutte en 20 minutes à une température comprise entre 20°C et 25°C à une suspension de 3,4 g d'hydrure de sodium à 80% dans 45 ml de diméthylformamide anhydre maintenue sous atmosphère d'azote. Après 15 minutes d'agitation, une solution de 26 g de 2-bromo-5 fluoro-1indanone dans 190 ml de diméthylformamide anhydre est ajoutée goutte à goutte en 10 minutes à la même température. Le mélange est agité pendant 1 heure 30 minutes puis, après addition lente de 100 ml d'eau, versé sur 3800 ml d'eau distillée et extrait 4 fois par 3800 ml au total de chloroforme. Les extraits organiques sont réunis, lavés avec 950 ml d'eau distillée, séchés sur du sulfate de sodium anhydre et concentrés à sec sous pression réduite (15 mm Hg; 2 kPa) à 40°C. Le produit obtenu (27 g) est chromatographié sur 1490 g de gel de silice neutre (0,020-0,045 mm) contenus dans une colonne de 9,6 cm de diamètre en éluant sous pression avec un mélange dichlorométhane-acétate d'éthyle (70-30 en volumes) et en recueillant des fractions de 80 ml. Les fractions 13 à 70 sont réunies et concentrées à sec sous pression réduite (15 mm Hg; 2 kPa) à 40°C. On obtient ainsi 13,4 g de 1-[2-(5-fluoro-1-oxo-indanyl)]imidazole-2-carboxylate d'éthyle fondant à 127°C.

La 2-bromo-5 fluoro-1-indanone peut être préparée de la façon suivante : une solution de 20,6 g de brome dans 90 ml d'acide acétique est ajoutée goutte à goutte en 1 heure à 20°C à une solution de 20 g de 5-fluoro-1-indanone et de 0,1 ml d'une solution aqueuse d'acide bromhydrique à 47% dans 260 ml d'acide acétique. Après 2 heures d'agitation, le mélange est versé sur 850 ml d'eau distillée et extrait 3 fois par 850 ml au total de chlorure de méthylène. Les extraits organiques sont réunis, lavés avec 200 ml d'eau distillée, séchés sur du sulfate de sodium anhydre et concentrés à sec sous pression réduite (15 mm Hg; 2 kPa) à 30°C. Le produit obtenu (31 g) est chromatographié sur 1860 g de gel de silice neutre (0,020-0.045 mm) contenus dans une colonne de 9,8 cm de diamètre en éluant avec un mélange cyclohexane-acétate d'éthyle (70-30 en volumes) et en recueillant une fraction de 10,5 litres qui est éliminée puis une fraction de 25 litres qui est concentrée sous pression réduite (15 mm Hg; 2 kPa) à 40°C. On obtient ainsi 28,4 g de 2-bromo-5 fluoro-1-indanone sous forme d'une huile jaune [Rf=0,7; chromatographie sur couche mince de gel de silice; solvant : cyclohexane-acétate d'éthyle (70-30 en volumes)].

### EXEMPLE 2

On opère comme dans l'exemple 1 mais à partir de 1,8 g de 1-[2-(5-méthyl-1-oxo-indanyl)]imidazole-2-carboxamide, de 80 ml au total de méthanol et de 22 ml de solution aqueuse d'acide chlorhydrique 12N. On obtient ainsi 1,5 g de chlorhydrate de 8-méthyl-5H,10H-imidazo[1,2-a]indéno[1,2-e]pyrazine-4-one se décomposant sans fondre au-dessus de 300°C [Spectre de R.M.N. : (200 MHz; DMSO d6; δ en ppm) : 2,42 (s, 3H: Ar-CH3); 4,05 (s, 2H : -CH2- en 10); 7,27 (d large, J=8 Hz 1H : -H7); 7,48 (s large, 1H : -H9); 7,83 (d, J=8 Hz, 1H : -H6); 7,97 et 8,25 (2d, J=1,5 Hz, 1H chacun : -H de l'imidazole); 13,20 (mf, 1H : -CONH-)].

Le 1-[2-(5-méthyl-1-oxo-indanyl)]imidazole-2-carboxamide peut être préparé comme dans l'exemple 1 pour la préparation du 1-[2-(5-fluoro-1-oxo-indanyl)]imidazole-2-carboxamide mais à partir de 2,4 g de 1-[2-(5-méthyl-1-oxo-indanyl)]imidazole-2-carboxylate d'éthyle et de 75 ml d'une solution 2,5 N de méthanol ammoniacal. On obtient ainsi 1,9 g de 1-[2-(5-méthyl-1-oxo-indanyl)]imidazole-2-carboxamide sous forme d'un solide fondant à 196°C.

Le 1-[2-(5-méthyl-1-oxo-indanyl)]imidazole-2-carboxylate d'éthyle peut être préparé comme dans l'exemple 1 pour la préparation du 1-[2-(5-fluoro-1-oxo-indanyl)]imidazole-2-carboxylate d'éthyle mais à partir de 2,24 g d'imidazole-2-carboxylate d'éthyle, de 0,6 g d'hydrure de sodium à 80% de 4,6 g de 2-bromo-5-méthyl-1-indanone et de 62 ml au total de diméthylformamide anhydre. Après chromatographie sur gel de silice avec un mélange dichlorométhane-acétate d'éthyle (80-20 en volumes), on obtient 2,5 g de 1-[2-(5-méthyl-1-oxo-indanyl)]imidazole-2-carboxylate d'éthyle fondant à 131°C.

La 2-bromo-5-méthyl-1-indanone peut être préparée comme dans l'exemple 1 pour la préparation de la 2-bromo-5 fluoro-1-indanone mais à partir de 7,3 g de 5-méthyl-1-indanone, de 8 g de brome, de 0,05 ml d'une solution aqueuse d'acide bromhydrique à 47% et de 165 ml au total d'acide acétique. Après chromatographie sur gel de silice avec un mélange cyclohexanedichlorométhane (70-30 en volumes), on obtient 4,6 g de 2-bromo-5-méthyl-1-indanone fondant à 54°C.

### EXEMPLE 3

On opère comme dans l'exemple 1 mais à partir de 2 g de 1-[2-(4-méthyl-1-oxo-indanyl)]imidazole-2-carboxamide, de 60 ml au total de méthanol et de 25 ml de solution aqueuse d'acide chlorhydrique 12N. On obtient ainsi 1,45 g de chlorhydrate de 9-méthyl-5H,10H-imidazo[1,2-a]indéno[1,2-e]pyrazine-4-one se décomposant sans fondre au-dessus de 300°C [Spectre de R.M.N. : (300 MHz; DMSO d6 plus quelques gouttes de CD3COOD d4; δ en ppm) : 2,40 (s, 3H : -CH3); 4,01 (s, 2H : -CH2- en 10); 7,23 (d, J=8 Hz 1H : -H8); 7,36 (t, J=8 Hz, 1H : -H7); 7,8 (d, J=8 Hz, 1H : -H6); 8,17 et 8,36 (2d, J=1 Hz, 1H chacun : -H de l'imidazole).

Le 1-[2-(4-méthyl-1-oxo-indanyl)]imidazole-2-carboxamide peut être préparé comme dans l'exemple 1 pour la préparation du 1-[2-(5-fluoro-1-oxo-indanyl)]imidazole-2-carboxamide mais à partir de 2,7 g de 1-[2-(4-méthyl-1-oxo-indanyl)]imidazole-2-carboxylate d'éthyle et de 160 ml d'une solution 2,5 N de méthanol ammoniacal. On obtient ainsi 2,1 g de 1-[2-(4-méthyl-1-oxo-indanyl)]imidazole-2-carboxamide sous forme d'un solide fondant à 160°C.

Le 1-[2-(4-méthyl-1-oxo-indanyl)]imidazole-2-carboxylate d'éthyle peut être préparé comme dans l'exemple 1 pour la préparation du 1-[2-(5-fluoro-1-oxo-indanyl)]imidazole-2-carboxylate d'éthyle mais à partir de 2,8 g d'imidazole-2-carboxylate d'éthyle, de 0,7 g d'hydrure de sodium à 80%, de 4,6 g de 2-bromo-4-méthyl-1-indanone et de 75 ml au total de diméthylformamide anhydre. Après chromatographie sur gel de silice avec un mélange dichlorométhane-acétate d'éthyle (80-20 en volumes), on obtient 2,8 g de 1-[2-(4-méthyl-1-oxo-indanyl)]imidazole-2-carboxylate d'éthyle fondant à 123°C.

La 2-bromo-4-méthyl-1-indanone peut être préparée comme décrit par K. MORI, Agr. Biol. Chem.,27(1), 22 (1963).

### EXEMPLE 4

On opère comme dans l'exemple 1 mais à partir de 1 g de 1-[2-(6-chloro-1-oxo-indanyl)]imidazole-2-carboxamide, de 75 ml au total de méthanol et de 15 ml de solution aqueuse d'acide chlorhydrique 12N. On obtient ainsi 0,4 g de chlorhydrate de 7-chloro-5H,10H-imidazo[1,2-a]indéno[1,2-e]pyrazine-4-one se décomposant sans fondre au-dessus de 300°C [Spectre de R.M.N. : (300 MHz; DMSO d6; δ en ppm) : 4,12 (s, 2H : -CH2- en 10); 7,44 (dd, J=8 et 1 Hz 1H : -H8); 7,66 (d, J=8 Hz, 1H : -H9); 7,95 et 8,25 (2s larges, 1H chacun : -H de l'imidazole); 8,05 (d, J=1 Hz, 1H : -H6); 12,97 (mf, 1H : -CONH-)].

Le 1-[2-(6-chloro-1-oxo-indanyl)]imidazole-2-carboxamide peut être préparé comme dans l'exemple 1 pour la préparation du 1-[2-(5-fluoro-1-oxo-indanyl)]imidazole-2-carboxamide mais à partir de 1,2 g de 1-[2-(6-chloro-1-oxo-indanyl)]imidazole-2-carboxylate d'éthyle et de 45 ml d'une solution 2,5 N de méthanol ammoniacal. On obtient ainsi 1 g de 1-[2-(6-chloro-1-oxo-indanyl)]imidazole-2-carboxamide sous forme d'un solide fondant à 190°C.

Le 1-[2-(6-chloro-1-oxo-indanyl)]imidazole-2-carboxylate d'éthyle peut être préparé comme dans l'exemple 1 pour la préparation du 1-[2-(5-fluoro-1-oxo-indanyl)]imidazole-2-carboxylate d'éthyle mais à partir de 2,5 g d'imidazole-2-carboxylate d'éthyle, de 0,7 g d'hydrure de sodium à 80%, de 5,4 g de 2-bromo-6-chloro-1-indanone et de 70 ml au total de diméthylformamide anhydre. Après chromatographie sur gel de silice avec un mélange dichlorométhane-acétate d'éthyle (70-30 en volumes), on obtient 1 g de 1-[2-(6-chloro-1-oxo-indanyl)]imidazole-2-carboxylate d'éthyle fondant à 180°C.

La 2-bromo-6-chloro-1-indanone peut être préparée comme décrit dans le brevet allemand 2 640 358.

### EXEMPLE 5

On opère comme dans l'exemple 1 mais à partir de 0,84 g de 1-[2-(5-chloro-1-oxo-indanyl)]imidazole-2-carboxamide, de 55 ml au total de méthanol et de 4 ml de solution aqueuse d'acide chlorhydrique 12N. On obtient ainsi 0,64 g de chlorhydrate de 8-chloro-5H,10H-imidazo[1,2-a]indéno[1,2-e]pyrazine-4-one se décomposant sans fondre au-dessus de 300°C [Spectre de R.M.N. : (200 MHz; DMSO d6; δ en ppm) : 4,07 [s, 2H: -CH2- en 10 (observé en DMSO d6 plus quelques gouttes de CD3COOD d4)]; 7,55 (dd, J=8 et 2 Hz 1H : -H7); 7,76 (d, J=2 Hz, 1H : -H9); 7,95 (d, J=8 Hz, 1H : -H6); 7,95 et 8,25 (2d, J=1.5 Hz, 1H chacun : -H de l'imidazole); 13,00 (mf, 1H : -CONH-)].

Le 1-[2-(5-chloro-1-oxo-indanyl)]imidazole-2-carboxamide peut être préparé de la façon suivante : une solution de 1,2 g de 1-[2-(5-chloro-1-oxo-indanyl)]imidazole-2-carboxylate d'éthyle dans 45 ml de méthanol est maintenue saturée pendant 6 heures à l'ébullition par un courant de gaz ammoniac. Après refroidissement à 5°C, les cristaux sont séparés par filtration, lavés avec 1 ml de méthanol glacé et séchés à l'air à 20°C. On obtient ainsi 0,4 g de 1-[2-(5-chloro-1-oxo-indanyl)]imidazole-2-carboxamide sous forme d'un solide fondant à 240°C.

Le 1-[2-(5-chloro-1-oxo-indanyl)]imidazole-2-carboxylate d'éthyle peut être préparé de la façon suivante : une solution de 3,65 g de 1,8-diazabicyclo[5.4.0]undec-7-ène dans 10 ml de chloroforme est additionnée goutte à goutte en 20 minutes à une température voisine de 20°C à une solution de 2,8 g d'imidazole-2-carboxylate d'éthyle et de 6,1 g de 2-bromo-5-chloro-1-indanone. Après 2 heures d'agitation et addition de 100 ml d'eau distillée, le mélange est extrait 3 fois avec 75 ml au total de chloroforme et les extraits organiques sont réunis, séchés sur du sulfate de magnésium anhydre et concentrés à sec sous pression réduite (15 mm Hg; 2 kPa) à 50°C. Après chromatographie sur gel de silice avec un mélange dichlorométhane-acétate d'éthyle (70-30 en volumes), on obtient 1 g de 1-[2-(5-chloro-1-oxo-indanyl)imidazole-2-carboxylate d'éthyle sous la forme d'une huile jaune épaisse [Rf= 0,3, chromatographie sur couche mince de gel de silice; solvant: dichlorométhane-acétate d'éthyle (70-30 en volumes)].

La 2-bromo-5-chloro-1-indanone peut être préparée comme décrit dans le brevet allemand 2 640 358.

### EXEMPLE 6

On opère comme dans l'exemple 1 mais à partir de 1,4 g de 1-[2-(4-chloro-1-oxo-indanyl)]imidazole-2-carboxamide, de 70 ml au total de méthanol et de 21 ml de solution aqueuse d'acide chlorhydrique 12N. On obtient ainsi 0,53 g de 9-chloro-5H,10H-imidazo[1,2-a]indéno[1,2-e]pyrazine-4-one se décomposant sans fondre au-dessus de 300°C [Spectre de R.M.N. : (200 MHz; DMSO d6; δ en ppm) : 4,06 (s, 2H : -CH2- en 10); 7,40 (dd, J=8 et 1 Hz 1H : -H8); 7,49 (t, J=8 Hz, 1H : -H7); 7,61 et 8,08 (2d, J=1 Hz, 1H chacun : -H de l'imidazole); 7,85 (d, J=8 Hz, 1H : -H6); 12,47 (mf, 1H, -CONH-)].

Le 1-[2-(4-chloro-1-oxo-indanyl)]imidazole-2-carboxamide peut être préparé de la façon suivante : une solution de 1,8 g de 1-[2-(4-chloro-1-oxo-indanyl)]imidazole-2-carboxylate d'éthyle dans 170 ml d'une solution 2,5N de méthanol ammoniacal est conservée pendant 16 heures à une température voisine de 20°C puis concentrée à sec sous pression réduite (15 mm Hg; 2 kPa) à 50°C. Le produit obtenu est mis en suspension dans 30 ml d'oxyde d'isopropyle, filtré, lavé 2 fois par 10 ml au total d'oxyde d'isopropyle puis séché sous pression réduite (15 mm Hg; 2 kPa) à une température voisine de 20°C. On obtient ainsi 1,4 g de 1-[2-(4-chloro-1-oxo-indanyl)]imidazole-2-carboxamide sous forme d'un solide fondant à 90°C.

Le 1-[2-(4-chloro-1-oxo-indanyl)]imidazole-2-carboxylate d'éthyle peut être préparé comme dans l'exemple 1 pour la préparation du 1-[2-(5-fluoro-1-oxo-indanyl)]imidazole-2-carboxylate d'éthyle mais à partir de 2,8 g d'imidazole-2-carboxylate d'éthyle, de 0,6 g d'hydrure de sodium à 80%, de 6,4 g de 2-bromo-4-chloro-1-indanone et de 80 ml au total de diméthylformamide anhydre. Après chromatographie sur gel de silice avec un mélange dichlorométhane- acétate d'éthyle (80-20 en volumes), on obtient 1,85 g de 1-[2-(4-chloro-1-oxo-indanyl)]imidazole-2-carboxylate d'éthyle [Rf=0,3, chromatographie sur couche mince de gel de silice; solvant : dichlorométhane-acétate d'éthyle (80-20 en volumes)].

La 2-bromo-4-chloro-1-indanone peut être préparée de la façon suivante: une solution de 16 g de brome dans 45 ml d'acide acétique est ajoutée goutte à goutte en 30 minutes à une température voisine de 20°C à une suspension de 17,5 g de 4-chloro-1-indanone dans un mélange de 240 ml d'acide acétique et de 0,1 ml d'une solution aqueuse d'acide bromhydrique à 47%. Après 2 heures d'agitation à la même température, le mélange est versé sur 380 ml d'eau distillée. Les cristaux sont séparés par filtration, lavés 2 fois avec 60 ml au total d'eau distillée et séchés sous pression réduite (15 mm Hg; 2 kPa) à 45°C. Le produit obtenu (19,3 g) est mis en suspension dans 60 ml d'éther de pétrole, filtré, lavé avec 10 ml d'éther de pétrole et séché sous pression réduite (15 mm Hg; 2 kPa) à 45°C. On obtient ainsi 14,7 g de 2-bromo-4-chloro-1-indanone fondant à 72°C.

La 4-chloro-1-indanone peut être préparée comme décrit dans le brevet américain 4 096 173.

### EXEMPLE 7

On opère comme dans l'exemple 1 mais à partir de 0,5 g de 1-[2-(4-phényl-1-oxo-indanyl)]imidazole-2-carboxamide, de 60 ml au total de méthanol et de 8 ml de solution aqueuse d'acide chlorhydrique 12N. On obtient ainsi 0,35 g de chlorhydrate de 9-phényl-5H,10H-imidazo[1,2-a] indéno[1,2-e]pyrazine-4-one se décomposant sans fondre au-dessus de 300°C [Spectre de R.M.N. : (300 MHz; DMSO d6; δ en ppm) : 4,16 (s, 2H: -CH2- en 10); 7,41 et 7,98 (2dd, J=8 et 1 Hz, 1H chacun : -H6 et -H8); 7,47 (t large, J=8 Hz, 1H : aromatique en para du phényl); 7,55 (t, J=8 Hz, 1H : -H7); 7,58 (t large, J=8 Hz, 2H : aromatiques en méta du phényl); 7,67 (d large, J=8 Hz, 2H : aromatiques en ortho du phényl); 7,91 et 8,26 (2d, J=1 Hz, 1H chacun : -H de l'imidazole); 13,00 (mf, 1H, -CONH-)].

Le 1-[2-(4-phényl-1-oxo-indanyl)]imidazole-2-carboxamide peut être préparé comme dans l'exemple 1 pour la préparation du 1-[2-(5-fluoro-1-oxo-indanyl)]imidazole-2-carboxamide mais à partir de 0,64 g de 1-[2-(5-méthyl-1-oxo-indanyl)]imidazole-2-carboxylate d'éthyle et de 80 ml d'une solution 5 N de méthanol ammoniacal. On obtient ainsi 0,5 g de 1-[2-(4-phényl-1-oxo-indanyl)]imidazole-2-carboxamide sous forme d'un solide fondant à 196°C.

Le 1-[2-(4-phényl-1-oxo-indanyl)]imidazole-2-carboxylate d'éthyle peut être préparé de la façon suivante : un mélange de 1,1 g d'imidazole-2-carboxylate d'éthyle et de 1,15 g de 2-bromo-4-phényl-1-indanone est chauffé pendant 20 minutes à 130°C, refroidi à 20°C et dissous dans 25 ml de dichlorométhane. La solution est lavée avec 10 ml d'eau distillée et, après décantation, la phase aqueuse est extraite 2 fois avec 20 ml au total de dichlorométhane. Les phases organiques sont réunies, lavées avec 10 ml d'une solution saturée d'hydrogénocarbonate de sodium, 2 fois avec 20 ml au total d'eau distillée, séchées sur du sulfate de sodium anhydre et concentrées a sec sous pression réduite (15 mm Hg; 2 kPa) à 35°C. Le produit obtenu (1,55 g) est chromatographié sur 155 g de gel de silice neutre (0,020-0,045 mm) contenus dans une colonne de 3,3 cm de diamètre en éluant sous pression avec un mélange dichlorométhane-acétate d'éthyle (80-20 en volumes) et en recueillant des fractions de 10 ml. Les fractions 72 à 140 sont réunies et concentrées à sec sous pression réduite (15 mm Hg; 2 kPa) à 40°C. On obtient ainsi 0,55 g de 1-[2-(4-phényl-1-oxo-indanyl)]imidazole-2-carboxylate d'éthyle fondant à 185°C.

La 2-bromo-4-phényl-1-indanone peut être préparée comme dans l'exemple 1 pour la préparation de la 2-bromo-5 fluoro-1-indanone mais à partir de 2,65 g de 4-phényl-1-indanone, de 2 g de brome, de 0,1 ml d'une solution aqueuse d'acide bromhydrique à 47% et de 55 ml au total d'acide acétique. Après chromatographie sur gel de silice avec un mélange cyclohexane-dichlorométhane (50-50 en volumes), on obtient 2,1 g de 2-bromo-4-phényl-1-indanone fondant à 98°C.

La 4-phényl-1-indanone peut être préparée comme décrit dans le brevet américain 4 263 319.

### EXEMPLE 8

On opère comme dans l'exemple 1 mais à partir de 0,46 g de 1-{2-[5-(1-imidazolyl)-1-oxo-indanyl}imidazole-2-carboxamide, de 35 ml au total de méthanol et de 7 ml de solution aqueuse d'acide chlorhydrique 12N. On obtient ainsi 0,23 g de chlorhydrate de 8-(1-imidazolyl)-5H,10H-imidazo[1,2-a]indéno[1,2-e]pyrazine-4-one se décomposant sans fondre au-dessus de 300°C [Spectre de R.M.N.: (200 MHz; DMSO d6; δ en ppm) : 4,18 (s, 2H : -CH2- en 10); 7,71 et de 8,00 à 8,15 (respectivement s large et mt, 1H pour le s : -H de l'imidazole); 7,88 (dd, J=8 et 2 Hz 1H : -H7); 7,96 et 8,34 (2 mt, 1H chacun : -H4' et -H5' de l'imidazole en 8); 8,08 (d, J=8 Hz : -H6); de 8,00 à 8,15 (mt: -H9); 9,73 (s large, 1H : -H2' de l'imidazole en 8); 12,65 (mf, 1H : -CONH-)].

Le 1-{2-[5-(1-imidazolyl)-1-oxo-indanyl}imidazole-2-carboxamide peut être préparé comme dans l'exemple 1 pour la préparation du 1-[2-(5-fluoro-1-oxo-indanyl)]imidazole-2-carboxamide mais à partir de 0,6 g de 1-{2-[5-(1-imidazolyl)-1-oxo-indanyl}imidazole-2-carboxylate d'éthyle et de 30 ml d'une solution 2,5 N de méthanol ammoniacal. On obtient ainsi 0,46 g de 1-{2-[5-(1-imidazolyl)-1-oxo-indanyl}imidazole-2-carboxamide sous la forme d'un solide fondant à 140°C.

Le 1-{2-[5-(1-imidazolyl)-1-oxo-indanyl)imidazole-2-carboxylate d'éthyle peut être préparé de la façon suivante : 1,1 g de brome est ajouté à 70°C à une solution de 1,2 g de 5-(1-imidazolyl)-1-indanone dans un mélange de 10 ml d'acide acétique et de 0,5 ml d'une solution aqueuse d'acide bromhydrique à 47%. Après 30 minutes d'agitation à la même température, le mélange est versé sur 50 ml d'eau distillée, neutralisé par addition de 15 g de carbonate de sodium et extrait 4 fois avec 90 ml au total de dichlorométhane. Les extraits organiques sont réunis, lavés avec 20 ml d'une solution aqueuse saturée de chlorure de sodium, séchés sur du sulfate de sodium anhydre, additionnés de 10 ml de diméthylformamide anhydre et le dichlorométhane est éliminé sélectivement sous pression réduite (15 mm Hg; 2 kPa) à 40°C. La solution dans le diméthylformamide ainsi obtenue (10 ml) est ajoutée goutte à goutte en 5 minutes à 25°C à une solution de 0,7 g d'imidazole-2-carboxylate d'éthyle dans 10 ml de diméthylformamide anhydre préalablement additionnée comme à l'exemple 1 de 0,7 g d'hydrure de sodium à 80%. Après 1 heure 30 minutes d'agitation à là même température, 5 ml d'eau distillée sont ajoutés lentement puis le mélange est versé sur 200 ml d'eau distillée et extrait 3 fois avec 180 ml au total de chloroforme. Les extraits organiques sont réunis, lavés avec 60 ml d'eau distillée, séchés sur du sulfate de sodium anhydre et concentrés à sec sous pression réduite (15mm Hg; 2 kPa) à 60°C. Le produit obtenu (1 g) est chromatographié sur 60 g de gel de silice neutre (0,020-0,045 mm) contenus dans une colonne de 2,9 cm de diamètre en éluant sous pression avec un mélange acétate d'éthyle-méthanol (85-15 en volumes) et en recueillant des fractions de 10 ml. Les fractions 24 à 56 sont réunies et concentrées à sec sous pression réduite (15 mm Hg; 2 kPa) à 40°C. On obtient ainsi 0,69 g de 1-{2-[5-(1-imidazolyl)-1-oxo-indanyl}imidazole-2-carboxylate d'éthyle [Rf=0,4, chromatographie sur couche mince de gel de silice; solvant : acétate d'éthyle-méthanol (80-20 en volumes)].

La 5-(1-imidazolyl)-1-indanone peut être préparée comme décrit dans le brevet européen 314 400.

### EXEMPLE 9

1 g de nitrate de potassium est ajouté en 10 minutes à une température voisine de 5°C à une solution de 2,6 g de chlorhydrate de 5H,10H-imidazo[1,2-a]indéno[1,2-e]pyrazine-4-one dans 20 ml d'acide sulfurique concentré (d= 1,83). Le mélange est agité pendant 30 minutes à la même température et pendant 3 heures à 25°C puis est versé sur 150 ml d'eau glacée. Les cristaux apparus sont séparés par filtration, lavés avec de l'eau distillée puis avec de l'acétone et séchés sous pression réduite (1 mm Hg; 0,13 kPa) à 80°C. On obtient ainsi 2,1 g de 8-nitro-5H,10H-imidazo[1,2-a]indéno[1,2-e]pyrazine-4-one se décomposant sans fondre au-dessus de 300°C [Spectre de R.M.N. : (200 MHz; DMSO d6; δ en ppm) : 4,23 (s, 2H : -CH2- en 10); 7,68 et de 8,12 (2s larges, 1H chacun : -H de l'imidazole); 8,07 (dd, J=8,5 Hz, 1H : -H6); 8,38 (dd, J=8,5 et 1,5 Hz, 1H : -H7); 8,50 (d, J=1,5 Hz, 1H: -H9); 12,64 (mf, 1H : -CONH-)].

### EXEMPLE 10

0,5 g de chlorhydrate de 5H,10H-imidazo[1,2-a]indéno[1,2-e]pyrazine-4-one est dissous dans 1,9 g d'acide chlorosulfonique à 5°C. La solution obtenue est agitée pendant 10 minutes à la même température et pendant 1 heure à 20°C puis est versée sur 50 g d'un mélange d'eau et de glace. L'insoluble apparu est séparé par filtration, lavé successivement 3 fois avec 30 ml au total d'eau distillée, 3 fois avec 30 ml au total d'acétone et avec 10 ml d'éther éthylique puis séché à l'air. Le produit obtenu (0,5 g) est dissous dans 50 ml d'une solution aqueuse de soude 2N puis le mélange est acidifié à pH 2 par addition d'une solution aqueuse d'acide chlorhydrique 1N. L'insoluble apparu estséparé par filtration, lavé successivement 3 fois avec 30 ml au total d'eau distillée, 3 fois avec 30 ml au total d'acétone et avec 10 ml d'éther éthylique puis séché sous pression réduite(1 mm Hg; 0,13 kPa) à 50°C. On obtient ainsi 0,45 g d'acide 4-oxo-5H,10H-imidazo[1,2-a]indéno[1,2-e]pyrazine-8-sulfonique se décomposant sans fondre au-dessus de 300°C [Spectre de R.M.N. : (300 MHz; DMSO d6; δ en ppm) : 4,10 (s, 2H : -CH2- en 10); 7,69 (dd, J=8 et 1 Hz 1H : -H7); 7,85 (d, J=1 Hz, 1H : -H9); 7,86 (d, J=8 Hz, 1H : -H6); 8,11 et 8,36 (2d, J=1 Hz, 1H chacun : -H de l'imidazole); 13,27 (mf, 1H : -CONH-)].

### EXEMPLE 11

Une solution de 2,3 g de bromure de 3,7-diméthyl-4-oxo-5H,10H-imidazo[1,2-a]indéno[1,2-e]pyrazinium dans 15 g d'imidazole est agitée sous atmosphère d'azote pendant 20 heures à 160°C, refroidie à 100°C puis versée sur 60 g d'un mélange d'eau distillée et de glace. L'insoluble est séparé par filtration, lavé 2 fois avec 10 ml au total d'eau distillée et avec 5 ml d'éther éthylique puis séché à l'air. Le produit (1,7 g) est chromatographié sur 100 g de gel de silice neutre (0,020-0,045 mm) contenus dans une colonne de 3,2 cm de diamètre en éluant sous pression avec un mélange dichlorométhane-méthanol (97-7 en volumes) et en recueillant des fractions de 10 ml. Les fractions 35 à 182 sont réunies et concentrées à sec sous pression réduite (15 mm Hg; 2 kPa) à 40°C. Le produit obtenu (0,6 g) est dissous dans un mélange bouillant de 64 ml de diméthylformamide et de 16 ml d'eau distillée et la solution, additionnée de noir décolorant, est filtrée à chaud, refroidie et conservée pendant 16 heures à 5°C. Les cristaux sont séparés par filtration, lavés avec 3 ml d'eau distillée et avec 3 ml d'éthanol puis séchés sous pression réduite (1 mm Hg; 0,13 kPa) à 60°C. On obtient ainsi 0,3 g de 7-méthyl-5H,10H-imidazo[1,2-a]indéno[1,2-e]pyrazine-4-one se décomposant sans fondre au-dessus de 300°C [Spectre de R.M.N. : (200 MHz; DMSO d6; δ en ppm) : 2,40 (s, 3H : Ar-CH3); 3,98 (s, 2H : -CH2- en 10); 7,15 (d large, J=8 Hz 1H : -H8); 7,49 (d, J=8 Hz, 1H : -H9); 7,60 et 7,99 (2s larges, 1H chacun : -H de l'imidazole); 7,72 (s large, 1H : -H6); 12,35 (mf, 1H : -CONH-)].

Le bromure de 3,7-diméthyl-4-oxo-5H,10H-imidazo[1,2-a]indéno[1,2-e] pyrazinium peut être préparé de la façon suivante : une solution de 4,75 g de 2-bromo-6-méthyl-1-indanone et de 2 g de 1-méthyl-1H-imidazole-2-carboxamide dans 40 ml de diméthylformamide anhydre est agitée pendant 16 heures à 115°C et refroidie à 20°C. Les cristaux sont séparés par filtration, lavés 2 fois avec 20 ml au total d'éther éthylique et séchés sous pression réduite (15 mm Hg; 2 kPa) à 50°C. On obtient ainsi 2,4 g de bromure de 3,7-diméthyl-4-oxo-5H,10H-imidazo[1,2-a]indéno[1,2-e] pyrazinium [Rf= 0,23, chromatographie sur couche mince de gel de silice, solvant : dichlorométhane-méthanol (8-2 en volumes)].

La 2-bromo-6-méthyl-1-indanone peut être préparée comme décrit par J. CHAKRAVARTY , Indian J. Chem., 7(3), 215 (1969).

### EXEMPLE 12

Une solution de 4,8 g de bromure de 3-méthyl-4-oxo-5H,10H-imidazo[1,2-a]indéno[1,2-e]pyrazinium dans 30 g d'imidazole est chauffée pendant 24 heures à 160°C, refroidie à 100°C puis versée sur un mélange agité de 75 g de glace et de 75 g d'eau distillée. L'insoluble est filtré, lavé 2 fois avec 20 ml au total d'eau distillée puis séché sous pression réduite (10 mm Hg; 1,3 kPa) à 50°C. Le produit ainsi obtenu (4 g) est dissous dans 80 ml de diméthylformamide et la solution additionnée de 20 g de silice est concentrée à sec sous pression réduite (15 mm Hg, 2 kPa) à 100°C. Le mélange est introduit dans une colonne de 4,2 cm de diamètre contenant 240 g de silice puis est élué par un mélange dichlorométhane-méthanol (97-3 en volumes) en recueillant des fractions de 60 ml. Les fractions 10 à 70 sont réunies, additionnées de 1,5 g de noir décolorant, filtrées et concentrées à sec sous pression réduite (15 mm Hg; 2 kPa) à 55°C. Le produit obtenu (1,7 g) est dissous dans 350 ml de méthanol bouillant et la solution additionnée de 0,1 g de noir décolorant est filtrée à chaud, concentrée sous pression réduite (15 mm Hg; 2 kPa) à 40°C pour ramener son volume à environ 30 ml puis conservée à 5°C pendant 60 heures. Les cristaux sont séparés par filtration, lavés 2 fois avec 20 ml au total de méthanol glacé et séchés sous pression réduite (1 mm Hg; 0,13 kPa) à 60°C. On obtient ainsi 1,1 g de 5H,10H-imidazo[1,2-a]indéno[1,2-e]pyrazine-4-one se décomposant sans fondre à 350°C [Rf= 0,77, chromatographie sur couche mince de gel de silice, solvant : dichlorométhane-méthanol (8-2 en volumes)].

Le bromure de 3-méthyl-4-oxo-5H,10H-imidazo[1,2-a]indéno[1,2-e] pyrazinium peut être préparé de la manière suivante : une solution de 5 g de 1-méthyl-1H-imidazole-2-carboxamide et de 12 g de 2-bromoindanone à 85% dans 100 ml de diméthylformamide anhydre est agitée pendant 28 heures à 115°C puis refroidie à une température voisine de 20°C. L'insoluble est séparé par filtration, lavé 2 fois avec 20 ml au total de diméthylformamide glacé et séché sous pression réduite (10 mm Hg; 1,3 kPa). On obtient ainsi 4,8 g de bromure de 3-méthyl-4-oxo-5H,10H-imidazo[1,2-a]indéno[1,2-e]pyrazinium utilisé tel quel dans les synthèses ultérieures [Spectre de R.M.N. : (200 MHz; DMSO d6; δ en ppm) : 4,13 (s, 2H: -CH2 en 10); 4,34 (s, 3H : N⁺ -CH3); 7,47 (mt, 2H : H7 et H8); 7,68 et 7,96 (2d, J = 7,5 Hz, 1H chacun : H6 et H9); 8,32 et 8,45 (2d, J = 1 Hz, 1H chacun : H de l'imidazole); 13,60 (mf, 1H : NH)].

La 1-méthyl-1H-imidazole-2-carboxamide peut être préparée selon le procédé décrit par D.D. DAVEY, J. Org. Chem., 52, 4379 (1987).

### EXEMPLE 13

On opère comme dans l'exemple 1 mais à partir de 3,8 g de 1-[2-(5-bromo-1-oxo-indanyl)]imidazole-2-carboxamide, de 420 ml au total de méthanol et de 57 ml de solution aqueuse d'acide chlorhydrique 12N. On obtient ainsi 3 g de chlorhydrate de 8-bromo-5H,10H-imidazo[1,2-a]indéno[1,2-e]pyrazine-4-one se décomposant sans fondre au-dessus de 300°C [Spectre de R.M.N.: (300 MHz; DMSO d6; δ en ppm) : 4,12 [s, 2H: -CH2- en 10 (observé en DMSO d6 plus quelques gouttes de CD3COOD d4)]; 7,68 (dd, J=8 et 2 Hz, 1H: -H7); 7,90 (s large, 1H: -H9); 7,93 (d, J=8 Hz, 1H: -H6); 8,02 et 8,32 (2d, J=1 Hz, 1H chacun: -H de l'imidazole); 13,18 (s large, 1H: -CONH-)].

Le 1-[2-(5-bromo-1-oxo-indanyl)]imidazole-2-carboxamide peut être préparé comme dans l'exemple 1 pour la préparation du 1-[2-(5-fluoro-1-oxo-indanyl)]imidazole-2-carboxamide mais à partir de 3,7 g de 1-[2-(5-bromo-1-oxo-indanyl)]imidazole-2-carboxylate d'éthyle et de 300 ml d'une solution 5 N de méthanol ammoniacal. On obtient ainsi 3,2 g de 1-[2-(5bromo-1-oxo-indanyl)]imidazole-2-carboxamide sous forme d'un solide fondant à 240°C.

Le 1-[2-(5-bromo-1-oxo-indanyl)]imidazole-2-carboxylate d'éthyle peut être préparé de la façon suivante : une solution de 9,5g d'imidazole-2-carboxylate d'éthyle, de 9,9 g de 2,5-dibromo-1-indanone et de 0,1 g d'iodure de sodium dans 280 ml d'éthanol est maintenue pendant 16 heures à l'ébullition, refroidie à 20°C et séchée sous pression réduite (20 mm Hg; 2,6 kPa) à 50°C. Le produit obtenu (21 g) est dissous dans 50 ml de dichlorométhane et la solution est lavée 3 fois avec 600 ml au total d'eau distillée, séchée sur du sulfate de magnésium anhydre et séchée sous pression réduite (20 mm Hg; 2,6 kPa) à 50°C. Le produit obtenu (12,7 g) est chromatographié sur 790 g de gel de silice neutre (0,020-0,045 mm) contenus dans une colonne de 7cm de diamètre en éluant sous pression avec un mélange dichlorométhane-acétate d'éthyle (70-30 en volumes) et en recueillant des fractions de 75 ml. Les fractions 70 à 140 sont réunies et concentrées à sec sous pression réduite ( 15 mm Hg; 2 kPa) à 40°C. On obtient ainsi 3,7 g de 1-[2-(5-bromo-1 -oxo-indanyl)]imidazole-2-carboxylate d'éthyle fondant à 140°C.

La 2,5-dibromo-1-indanone peut être préparée comme décrit dans le brevet européen 346107.

### EXEMPLE 14

420 mg de 1-[2-(4-fluoro-1-oxo-indanyl)]imidazole-2-carboxamide sont dissous dans 20 ml de méthanol bouillant et la solution, additionnée de 0,2 g de noir décolorant, est filtrée à chaud, concentrée sous pression réduite (15 mm Hg; 2kPa) à 40°C. Au résidu ainsi obtenu, on ajoute 6 ml d'acide chlorhydrique 12N et la solution est conservée à 5°C pendant 60 heures. Les cristaux sont séparés par filtration, redissous dans 20 ml de méthanol bouillant. Après addition de 20 ml d'éther chlorhydrique 6N et filtration, on obtient 130 mg de 9-fluoro-5H,10H-imidazo[1,2-a]indéno[1,2-e]pyrazine-4-one sous forme de chlorhydrate fondant à une température supérieure à 260°C (Analyse % calculé C : 56,23, H : 3,27, Cl : 12,77, F: 6,84, N : 15,13, % trouvé C : 56,20, H : 3,40, Cl : 13,00, F : 5,90, N : 14,40).

Le 1-[2-(4-fluoro-1-oxo-indanyl)]imidazole-2-carboxamide peut être obtenu de la manière suivante : 720 mg de 1-[2-(4-fluoro-1-oxo-indanyl)]imidazole-2-carboxylate d'éthyle sont dissous dans 50 ml de solution 3N de méthanol ammoniacal et la solution est maintenue sous agitation pendant 20 heures à une température voisine de 20°C, puis concentrée à sec sous pression réduite (15 mm Hg; 2kPa) à 40°C. Le résidu ainsi obtenu est purifié par flash-chromatographie sur colonne de silice, sous courant d'azote à moyenne pression (0,5 bar) avec de l'acétate d'éthyle comme éluant. On obtient ainsi 420 mg de 1-[2-(4-fluoro-1-oxo-indanyl)]imidazole-2-carboxamide fondant à 159°C.

Le 1-[2-(4-fluoro-1-oxo-indanyl)]imidazole-2-carboxylate d'éthyle peut être obtenu de la manière suivante : une solution de 5,3 g d' imidazole-2-carboxylate d'éthyle dans 20 ml de diméthylformamide anhydre est additionnée goutte à goutte en 30 minutes à une température voisine de 20°C à une suspension de 2,43 g d'hydrure de sodium à 50% dans 60 ml de diméthylformamide anhydre maintenue sous atmosphère d'azote. Après 15 minutes d'agitation, une solution de 9,26 g de 2-bromo-4-fluoro-1-indanone dans 25 ml de diméthylformamide anhydre est ajoutée goutte à goutte en 30 minutes à une température voisine de 20°C. Le mélange est agité pendant 18 heures, puis après addition lente de 30 ml d'eau, versé dans 200 ml d'eau distillée et extrait quatre fois par 100 ml de dichlorométhane. Les phases organiques sont réunies, lavées par 600 ml d'eau distillée, séchées sur sulfate de sodium anhydre et concentrées à sec sous pression réduite (15 mm Hg; 2kPa) à 45°C. Le résidu ainsi obtenu est purifié par flash-chromatographie sur colonne de silice, sous courant d'azote à moyenne pression (0,5 bar) avec de l'acétate d'éthyle comme éluant. On obtient ainsi 1,2 g de 1-[2-(4-fluoro-1-oxo-indanyl)]imidazole-2-carboxylate d'éthyle fondant à 145°C.

La 2-bromo-4-fluoro-1-indanone peut être obtenue de la manière suivante : à une solution de 8 g de 4-fluoro-1-indanone dans 400 ml de dioxanne, sont ajoutés 31,3 g de bromure de cuivre. La solution est ensuite portée à une température voisine de 100°C pendant 4 heures. Le milieu réactionnel est ensuite filtré sur célite, puis concentré à sec sous pression réduite (15 mm Hg; 2kPa) à 40°C. On obtient ainsi 13,23 g de 2-bromo-4-fluoro-1-indanone, utilisée telle quelle dans les synthèses ultérieures.

La 4-fluoro-1-indanone peut être préparée comme décrit par M.OLIVIER et E. MARECHAL, Bull. Soc. Chim. Fr.,(11), 3042 (1973).

### EXEMPLE 15

On opère comme dans l'exemple 14 mais à partir de 0,26 g de 1-[2-(6,7-dichloro-1-oxo-indanyl)]imidazole-2-carboxamide, de 80 ml de méthanol et de 20 ml d'acide chlorhydrique (12N). On obtient ainsi 97 mg de 6,7-dichloro-5H,10H-imidazo[1,2-a]indéno[1,2-e]pyrazine-4-one sous forme de chlorhydrate fondant à une température supérieure à 260°C (Analyse % calculé C : 49,35, H : 2,44, Cl : 29,88, N : 13,28, % trouvé C : 49,40, H : 2,10, Cl : 29,90, N : 13,10).

Le 1-[2-(6,7-dichloro-1-oxo-indanyl)]imidazole-2-carboxamide peut être préparé comme à l'exemple 14 pour la préparation du 1-[2-(4-fluoro-1-oxo-indanyl)]imidazole-2-carboxamide mais à partir de 1 g de 1-[2-(6,7-dichloro-1-oxo-indanyl)]imidazole-2-carboxylate d'éthyle et de 56 ml d'une solution 3N de méthanol ammoniacal. On obtient ainsi 270 mg de 1-[2-(6,7-dichloro-1-oxo-indanyl)]imidazole-2-carboxamide fondant à 270°C.

Le 1 -[2-(6,7-dichloro-1-oxo-indanyl)]imidazole-2-carboxylate d'éthyle peut être préparé comme à l'exemple 14 pour la préparation du 1-[2-(4-fluoro-1-oxo-indanyl)]imidazole-2-carboxylate d'éthyle mais à partir de 2,5 g d'imidazole-2-carboxylate d'éthyle, de 0,65 g d'hydrure de sodium à 80% , de 6,06 g de 2-bromo-6,7-dichloro-1-ihdanone et de 36 ml au total de diméthylformamide anhydre .Après purification par chromatographie sur colonne de silice avec l'acétate d'éthyle comme éluant, on obtient 1 g de 1-[2-(6,7-dichloro-1-oxo-indanyl)]imidazole-2-carboxylate d'éthyle fondant à 90°C.

La 2-bromo-6,7-dichloro-1-indanone est préparée comme dans l'exemple 14 pour la préparation de la 2-bromo-4-fluoro-1-indanone mais à partir de 7,42 g de 6,7-dichloro-1-indanone, de 21,8 g de bromure de cuivre et de 240 ml de dioxanne. Après purification par chromatographie sur colonne de silice avec un mélange acétate d'éthyle-cyclohexane (10-90 en volumes) comme éluant, on obtient 6,79 g de 2-bromo-6,7-dichloro-1-indanone fondant à 103°C.

La 6,7-dichloro-1-indanone peut être préparée de la manière suivante : 58,8 g d'acide 3-(3,4-dichlorophényl)propionique et 315 ml de chlorure de thionyle sont portés au reflux pendant 45 minutes. Après avoir éliminé par distillation l'excès de chlorure de thionyle, on ajoute 1200 ml de dichlorométhane anhydre et 47 g de chlorure d'aluminium. Le milieu réactionnel est porté au reflux pendant 6 heures, refroidi et on ajoute 1366 ml d'une solution aqueuse d'acide chlorhydrique 1N. Après décantation, la phase organique est lavée avec 1700 ml d'une solution aqueuse de potasse (5%) et 600 ml d'eau distillée, séchée sur sulfate de magnésium anhydre et concentrée à sec sous pression réduite (15 mm Hg; 2kPa) à 40°C. Le résidu ainsi obtenu est purifié par flash-chromatographie sur colonne de silice sous courant d'azote à moyenne pression (0,5 bar) avec un mélange acétate d'éthyle-cyclohexane (20-80) comme éluant. On obtient ainsi 7,42 g de 6,7-dichloro-1-indanone fondant à 129°C et 21,8 g de 5,6-dichloro-1-indanone fondant à 150°C.

L'acide 3-(3,4-dichlorophényl)propionique peut être préparé de la manière suivante : à une solution de 69,86 g d'acide 3,4-dichlorocinnamique, de 1750 ml de méthanol et de 21 g de chlorure de Nickel héxahydraté, on ajoute en 75 minutes, en maintenant la température voisine de 20°C, 57,75 g d'hydroborure de sodium. Après une heure d'agitation à une température voisine de 20°C, la solution est filtrée puis concentrée à sec sous pression réduite (15 mm Hg; 2kPa) à 40°C. Le résidu ainsi obtenu est dissous dans 875 ml d'une solution aqueuse de potasse (5%) et extrait par 1000 ml d'éther éthylique. La phase aqueuse est acidifiée par 945 ml d'une solution aqueuse 2N d'acide chlorhydrique puis extraite par deux fois 1000 ml d'éther éthylique. La phase organique est séchée sur sulfate de magnésium anhydre, concentrée à sec sous pression réduite (15 mm Hg; 2kPa) à 40°C et on obtient 58,8 g d'acide 3-(3,4-dichlorophényl)propionique fondant à 90°C.

### EXEMPLE 16

On opère comme à l'exemple 14 mais à partir de 0,11 g de 1-[2-(7,8-dichloro-1-oxo-indanyl)]imidazole-2-carboxamide, de 10 ml de méthanol et de 35 ml d'acide chlorhydrique (12N). On obtient ainsi 30 mg de 7,8-dichloro-5H,10H-imidazo[1,2-a]indéno[1,2-e]pyrazine-4-one sous forme de chlorhydrate fondant à une température supérieure à 260°C [Spectre infra-rouge (KBr) bandes caractéristiques en cm⁻¹ : 3425, 3200 à 2125, 2000, 3125, 3140, 3020, 1710 et épaulement à 1695, 1645, 1550, 1500, 1450, 1385, 1105, 780 et 675].

Le 1-[2-(7,8-dichloro-1-oxo-indanyl)]imidazole-2-carboxamide est préparé comme à l'exemple 14 pour la préparation du 1-[2-(4-fluoro-1-oxo-indanyl)]imidazole-2-carboxamide mais à partir de 0,18 g de 1-[2-(7,8-dichloro-1-oxo-indanyl)]imidazole-2-carboxylate d'éthyle et de 10 ml d'une solution 3N de méthanol ammoniacal. On obtient ainsi 0,13 mg de 1-[2-(7,8-dichloro-1-oxo-indanyl)]imidazole-2-carboxamide fondant à 264°C.

Le 1-[2-(7,8-dichloro-1-oxo-indanyl)]imidazole-2-carboxylate d'éthyle est préparé comme à l'exemple 14 pour la préparation du 1-[2-(4-fluoro-1-oxo-indanyl)]imidazole-2-carboxylate d'éthyle mais à partir de 1,66 g d' imidazole-2-carboxylate d'éthyle, de 0,47 g d'hydrure de sodium à 80%, de 4 g de 2-bromo-5,6-dichloro-1-indanone et de 24 ml au total de diméthylformamide anhydre. Après purification par chromatographie sur colonne de silice avec un mélange acétate d'éthyle-dichlorométhane (40-60 en volumes) comme éluant, on obtient 0,2 g de 1-[2-(7,8-dichloro-1-oxo-indanyl)]imidazole-2-carboxylate d'éthyle fondant à 103°C.

La 2-bromo-5,6-dichloro-1-indanone est préparée comme dans l'exemple 14 pour la préparation de la 2-bromo-4-fluoro-1-indanone mais à partir de 5,48 g de 5,6-dichloro-1-indanone, de 16,1 g de bromure de cuivre et de 175 ml de dioxanne. Après purification par chromatographie sur colonne de silice avec un mélange acétate d'éthyle-cyclohexane (10-90 en volumes) comme éluant, on obtient 4,07 g de 2-bromo-5,6-dichloro-1-indanone fondant à 85°C.

### EXEMPLE 17

On opère comme à l'exemple 14 mais à partir de 0,72 g de 1-[2-(8,9-dichloro-1-oxo-indanyl)]imidazole-2-carboxamide, de 220 ml de méthanol et de 55 ml d'acide chlorhydrique (12N). On obtient ainsi 0,57 g de 8,9-dichloro-5H,10H-imidazo[1,2-a]indéno[1,2-e]pyrazine-4-one sous forme de chlorhydrate fondant à une température supérieure à 260°C (Analyse % calculé C : 53,45, H : 2,42, Cl : 24,27, N : 14,38, O : 5,4, % trouvé C : 53,50, H : 2,50, Cl : 23,50, N : 14,40, O : 5,8).

Le 1-[2-(8,9-dichloro-1-oxo-indanyl)]imidazole-2-carboxamide peut être préparé comme à l'exemple 14 pour la préparation du 1-[2-(4-fluoro-1-oxo-indanyl)]imidazole-2-carboxamide mais à partir de 1,77 g de 1-[2-(8,9-dichloro-1-oxo-indanyl)]imidazole-2-carboxylate d'éthyle et de 95 ml d'une solution 3N de méthanol ammoniacal. On obtient ainsi 0,66 g de 1-[2-(8,9-dichloro-1-oxo-indanyl)]imidazole-2-carboxamide fondant à 202°C.

Le 1-[2-(8,9-dichloro-1-oxo-indanyl)]imidazole-2-carboxylate d'éthyle peut être préparé de la manière suivante : un mélange de 4,2 g d' imidazole-2-carboxylate d'éthyle et de 4,2 g de 2-bromo-4,5-dichloro-1-indanone est chauffé à 130°C pendant 20 minutes, refroidi à 20°C et dissous dans 90 ml de dichlorométhane. La solution est lavée par 35 ml d'eau. La phase organique est lavée avec 45 ml d'une solution aqueuse saturée de bicarbonate de sodium, 135 ml d'eau distillée, séchée sur sulfate de sodium anhydre et concentrée à sec sous pression réduite (15 mm Hg; 2kPa) à 40°C. Le résidu ainsi obtenu est purifié par flash-chromatographie sur colonne de silice avec un mélange acétate d'éthyle-cyclohexane (60-40 en volumes) comme éluant. On obtient ainsi 1,28 g de 1-[2-(8,9-dichloro-1-oxo-indanyl)]imidazole-2-carboxylate d'éthyle fondant à 158°C.

La 2-bromo-4,5-dichloro-1-indanone peut être préparée comme dans l'exemple 14 pour la préparation de la 2-bromo-4-fluoro-1-indanone mais à partir de 12,67 g de 4,5-dichloro-1-indanone, de 37,3 g de bromure de cuivre et de 400 ml de dioxanne. On obtient 16,79 g de 2-bromo-6,7-dichloro-1-indanone fondant à 131°C.

La 4,5-dichloro-1-indanone est préparée comme dans l'exemple 15 pour la préparation de la 6,7-dichloro-1-indanone mais à partir de 26 g d'acide 3 (2,3-dichlorophényl)propionique, de 138 ml de chlorure de thionyle, de 550 ml de dichlorométhane anhydre et de 20,5 g de chlorure d'aluminium. Après recristallisation dans 100 ml d'éthanol bouillant, on obtient ainsi 12,67 g de 4,5-dichloro-1-indanone fondant à 78°C.

L'acide 3-(2,3-dichlorophényl)propionique peut être préparé comme dans l'exemple 15 pour la préparation de l'acide 3-(3,4-dichlorophényl)propionique mais à partir de 33 g d'acide 2,3-dichlorocinnamique, de 800 ml de méthanol et de 9,9 g de chlorure de Nickel hexahydraté et de 27,22 g d'hydroborure de sodium. On obtient 26,33 g d'acide 3-(2,3-dichlorophényl)propionique fondant à 100°C.

### EXEMPLE 18

On opère comme à l'exemple 14 mais à partir de 1,27 g de 1-[2-(7,9-dichloro-1-oxo-indanyl)]imidazole-2-carboxamide, de 390 ml de méthanol et de 96 ml d'acide chlorhydrique (12N). On obtient ainsi 1,44 g de 7,9-dichloro-5H,10H-imidazo[1,2-a]indéno[1,2-e]pyrazine-4-one sous forme de chlorhydrate fondant à une température supérieure à 260°C (Analyse % calculé C : 47,52 , H : 2,45, Cl : 32,37, N : 12,79, O : 4,87, % trouvé C : 47,20, H : 2,30, Cl : 32,10, N : 12,70, O : 4,80).

Le 1-[2-(7,9-dichloro-1-oxo-indanyl)]imidazole-2-carboxamide peut être préparé comme à l'exemple 14 pour la préparation du 1-[2-(4-fluoro-1-oxo-indanyl)]imidazole-2-carboxamide mais à partir de 2,60 g de 1-[2-(7,9-dichloro-1-oxo-indanyl)]imidazole-2-carboxylate d'éthyle et de 145 ml d'une solution 3N de méthanol ammoniacal. On obtient ainsi 1,30 g de 1-[2-(7,9-dichloro-1-oxo-indanyl)]imidazole-2-carboxamide fondant à une température supérieure à 260°C (Rf= 0,49, chromatographie sur couche mince de gel de silice, solvant: acétate d'éthyle].

Le 1-[2-(7,9-dichloro-1-oxo-indanyl)]imidazole-2-carboxylate d'éthyle peut être préparé de la manière suivante : un mélange de 7,25 g d' imidazole-2-carboxylate d'éthyle et de 7,25 g de 2-bromo-4,6-dichloro-1-indanone est chauffé à 130°C pendant 30 minutes, refroidi à 20°C et dissous dans 100 ml de dichlorométhane. La solution est lavée par 200 ml d'eau. La phase organique est lavée avec 200 ml d'une solution aqueuse saturée de bicarbonate de sodium, 240 ml d'eau distillée, séchée sur sulfate de sodium anhydre et concentrée à sec sous pression réduite (15 mm Hg; 2kPa) à 40°C. Le résidu ainsi obtenu est purifié par flash-chromatographie sur colonne de silice avec un mélange acétate d'éthyle-cyclohexane (80-20 en volumes) comme éluant.On obtient ainsi 2,90 g de 1-[2-(7,9-dichloro-1-oxo-indanyl)]imidazole-2-carboxylate d'éthyle fondant à 127°C.

La 2-bromo-4,6-dichloro-1-indanone est préparée comme dans l'exemple 14 pour la préparation de la 2-bromo-4-fluoro-1-indanone mais à partir de 21,6 g de 4,6-dichloro-1-indanone, de 63,56 g de bromure de cuivre et de 690 ml de dioxanne. On obtient 14,5 g de 2-bromo-4,6-dichloro-1-indanone fondant à 80°C.

La 4,6-dichloro-1-indanone peut être préparée comme dans l'exemple 15 pour la préparation de la 6,7-dichloro-1-indanone mais à partir de 53 g d'acide 3-(2,4-dichlorophényl)propionique, de 284 ml de chlorure de thionyle, de 1130 ml de dichlorométhane anhydre et de 42,16 g de chlorure d'aluminium. On obtient après recristallisation dans 150 ml d'éthanol bouillant 21,62 g de 4,6-dichloro-1-indanone fondant à 120°C.

L'acide 3-(2,4-dichlorophényl)propionique peut être préparé comme dans l'exemple 15 pour la préparation de l'acide 3-(3,4-dichlorophényl)propionique mais à partir de 66 g d'acide 2,4-dichlorocinnamique, de 1600 ml de méthanol, de 19,8 g de chlorure de nickel hexahydraté et de 54,44 g d'hydroborure de sodium. On obtient ainsi 53,44 g d'acide 3-(2,4-dichlorophényl)propionique fondant à 83°C.

### EXEMPLE 19

1 g de 9-bromo-5H,10H-imidazo[1,2-a]indéno[1,2-e]pyrazine-4-one est ajouté à 6 ml d'acide sulfurique concentré (20N) à une température voisine de 0°C. 0,3 g de nitrate de potassium sont alors ajoutés en deux fois à cette solution tout en conservant la température entre 0 et 5°C. Le milieu réactionnel est agité pendant 18 heures à 20°C, puis versé dans 50 ml d'eau glacée. L'insoluble est filtré, lavé avec 50 ml d'acétate d'éthyle et séché sous pression réduite (15 mm Hg; 2kPa) à 50°C. On obtient ainsi 0,77 g de 8-nitro 9-bromo-5H,10H-imidazo[1,2-a]indéno[1,2-e]pyrazine-4-one fondant au-dessus de 260°C (Analyse % calculé C : 44,98, H : 2,03, Br : 23,02, N : 16,14, % trouvé C : 45,40, H : 2,20, Br : 21,30, N : 15,70).

La 9-bromo-5H,10H-imidazo[1,2-a]indéno[1,2-e]pyrazine-4-one peut être préparée de la manière suivante : 4,6 g de 1-[2-(4-bromo-1-oxo-indanyl)]imidazole-2-carboxamide sont dissous dans 350 ml de méthanol bouillant et la solution, additionnée de 0,2 g de noir décolorant, est filtrée à chaud, concentrée sous pression réduite (15 mm Hg; 2kPa) à 40°C. Au résidu ainsi obtenu, on ajoute 150 ml d'acide chlorhydrique 12N et la solution est conservée à 5°C pendant 18 heures. Les cristaux sont séparés par filtration, redissous dans 150 ml de méthanol bouillant. Après addition de 170 ml d'acide chlorhydrique 12N, le milieu est chauffé à 80°C pendant une heure. Après filtration, on obtient 3,45 g de 9-bromo-5H,10H-imidazo[1,2-a] indéno[1,2-e]pyrazine-4-one sous forme de chlorhydrate fondant à une température supérieure à 260°C (Analyse % calculé C: 46,11, H : 2,68, Br : 23,60, Cl : 10,47, N : 12,41, O : 4,73, % trouvé C : 46,20, H : 2,60, Br : 23,8, Cl : 9,90, N : 12,30, O : 4,80).

Le 1-[2-(4-bromo-1-oxo-indanyl)]imidazole-2-carboxamide peut être obtenu de la manière suivante : une solution de 3,5 g de 1-[2-(4-bromo-1-oxo-indanyl)]imidazole-2-carboxylate d'éthyle dans 20 ml de méthanol est maintenue saturée pendant une heure à l'ébullition par un courant de gaz ammoniac. Le milieu réactionnel est ensuite concentré à sec sous pression réduite (15 mm Hg; 2kPa) à 40°C. On obtient ainsi 3,10 g de 1-[2-(4-bromo-1-oxo-indanyl)]imidazole-2-carboxamide fondant à 212°C.

Le 1-[2-(4-bromo-1-oxo-indanyl)]imidazole-2-carboxylate d'éthyle peut être obtenu de la manière suivante : un mélange de 9,28 g d'imidazole-2-carboxylate d'éthyle et de 8,50 g de 2,4-dibromo-1-indanone est chauffé à 130°C pendant 20 minutes, refroidi à 20°C et dissous dans 50 ml de dichlorométhane. La solution est lavée par deux fois 100 ml d'eau. La phase organique est lavée avec 50 ml d'une solution aqueuse 0,1N de soude, 300 ml d'eau distillée, séchée sur sulfate de sodium anhydre et concentrée à sec sous pression réduite (15 mm Hg; 2kPa) à 40°C.Le résidu ainsi obtenu est purifié par flash-chromatographie sur colonne de silice, sous courant d'azote à moyenne pression (0,5 bar) avec de l'acétate d'éthyle comme éluant. On obtient ainsi 3,5 g de 1-[2-(4-bromo-1-oxo-indanyl)]imidazole-2-carboxylate d'éthyle fondant à 157°C.

La 2,4-dibromo-1-indanone peut être obtenue de la manière suivante : une solution de 24 g de 4-bromo-1-indanone et de 120 ml de chloroforme est refroidie à 5°C. Une solution de 18,2 g de brome et de 20 ml de chloroforme est alors ajoutée goutte à goutte en deux heures à une température comprise entre 0 et 5°C. Après avoir laissé le milieu réactionnel sous agitation pendant une heure en conservant la température d'introduction, on laisse revenir la solution à température ambiante et on agite encore une heure. Le milieu réactionnel est ensuite concentré à sec sous pression réduite (15 mm Hg; 2kPa) à 40°C. Le résidu ainsi obtenu est purifié par flash-chromatographie sur colonne de silice, sous courant d'azote à moyenne pression (0,5 bar) avec un mélange acétate d'éthyle-cyclohexane (10-90 en volumes) comme éluant.On obtient ainsi 13,63 g de 2,4-dibromo-1-indanone fondant à 80°C.

La 4-bromo-1-indanone peut être préparée comme décrit par F. G. HOLLIMAN, F. G. MANNE et D. A. THORNTON, J.Chem.Soc.,9 (1960).

### EXEMPLE 20

On dissout 6,3 g de 1-[2-(5-méthoxy-1-oxo-indanyl)]imidazole-2-carboxylate d'éthyle dans une solution 4 N d'acétate d'ammonium dans de l'acide acétique glacial. Après 12 heures de reflux, le mélange est refroidi à une température voisine de 20°C et filtré. Le solide obtenu est lavé à l'eau jusqu'à pH neutre et avec 50 ml d'acétone, puis il est recristallisé à chaud dans 75 ml de diméthylformamide. Les cristaux formés sont séparés par filtration et rincés successivement par 50 ml d'eau et 50 ml d'acétone. On obtient ainsi 3,3 g de 5H,10H-8-méthoxy-imidazo[1,2-a]indéno[1,2-e]pyrazine-4-one sous forme d'un solide beige fondant au-dessus de 260°C [Spectre de R.M.N. : (200 MHz; DMSO d6; δ en ppm) : 3,82 (s, 3H : -OCH3); 3,98 (s, 2H : -CH2- en 10); 6,98 (dd, J=8 et 1,5 Hz, 1H : -H7); 7,22 (d, J=1,5 Hz, 1H : -H9); 7,56 et 7,97 (2s, 1H chacun : -H de l'imidazole); 7,78 (d, J=8 Hz, 1H : -H6); 12,30 (s large, 1H: -CO-NH-)].

Le 1-[2-(5-méthoxy-1-oxo-indanyl)]imidazole-2-carboxylate d'éthyle peut être préparé de la façon suivante : un mélange de 13,9 g de 2-bromo-5-méthoxy-1-indanone et de 16,2 g de 2-éthoxycarbonylimidazole dans 300 ml de toluène est porté au reflux pendant 9 heures. Le toluène est alors évaporé sous pression réduite et le résidu est repris par du dichlorométhane et de l'eau. La phase organique est extraite avec du dichlorométhane et lavée à l'eau. Après traitement habituel, le produit brut est purifié par deux chromatographies successives sur colonne de silice avec respectivement des mélanges de dichlorométhane et d'acétate d'éthyle (70/30 en volumes) et de dichlorométhane et de méthanol (98/2 en volumes). On obtient ainsi 7,5 g de 1-[2-(5-méthoxy-1-oxo-indanyl)]imidazole-2-carboxylate d'éthyle (Rf= 0,38, .chromatographie sur couche mince de gel de silice, éluant : dichlorométhane-acétate d'éthyle (70-30 en volumes)).

La 2-bromo-5-méthoxy-1-indanone peut être synthétisée comme décrit par D. MUKHOPADHYA, D. N. CHAUDHURY, J. Indian Chem. Soc.,47(5), 450 (1970).

### EXEMPLE 21

Un mélange de 9,7 g de 5H,10H-8-nitro-imidazo[1,2-a]indéno[1,2-e]pyrazine-4-one, de 370 ml de soude aqueuse 0,1 N et de 0,3 g de charbon palladié à 10 % est hydrogéné à une température voisine de 20°C sous une pression de 1,2 bar pendant 23 heures. La suspension est acidifiée par 80 ml d'acide chlorhydrique 1 N, puis elle est filtrée. On reprend le solide obtenu dans 600 ml d'eau bouillante. Le mélange est additionné de noir animal et filtré à chaud sur cellite. Le filtrat cristallise après refroidissement dans un bain de glace. Les cristaux sont séparés par filtration et lavés deux fois avec 50 ml d'éther éthylique. On obtient ainsi 4,7 g de monochlorhydrate de 5H,10H-8-amino-imidazo[1,2-a]indéno[1,2-e]pyrazine-4-one sous forme d'un solide beige fondant au-dessus de 260°C [Spectre de R.M.N. : (300 MHz; DMSO d6; δ en ppm) : 4,02 (s, 2H : -CH2- en 10); 7,00 (d large, J=8 Hz, 1H : -H7); 7,20 (s large, 1H : -H9); 7,74 (d, J=8 Hz, 1H : -H6); 7,77 et 8,07 (2s larges, 1H chacun : -H de l'imidazole); 12,65 (mf, 1H : -CO-NH-)].

### EXEMPLE 22

On ajoute successivement 1,7 g de triéthylamine et 1,95 g de phénylisocyanate à une suspension de 1,5 g de monochlorhydrate de 5H,10H-8-amino-imidazo[1,2-a]indéno[1,2-e]pyrazine-4-one dans 50 ml de diméthylsulfoxyde. Le mélange est agité à une température voisine de 20°C pendant 15 heures, puis on le verse dans 150 ml d'eau glacée. Le précipité formé est séparé par filtration et cristallisé à chaud dans 20 ml de diméthylformamide. Les cristaux sont filtrés et lavés deux fois avec 50 ml d'éther éthylique. On obtient ainsi 1,4 g de 5H,10H-8-(3-phényluréido)-imidazo[1,2-a]indéno[1,2-e]pyrazine-4-one sous forme d'un solide beige rosé fondant au-dessus de 260°C [Spectre de R.M.N. : (200 MHz; DMSO d6; δ en ppm) : 4,01 (s large, 2H : -CH2- en 10); 7,01 (t large, J=7,5 Hz, 1H : -H aromatique en para du -NH-CO-NH-); 7,32 (t large, J=7,5 Hz, 2H : -H aromatiques en méta du -NH-CO-NH-); 7,38 (dd, J=8 et 1 Hz, 1H : -H7); 7,50 (d large, J=7,5 Hz, 2H : -H aromatiques en ortho du -NH-CO-NH-); 7,58 et 7,95 (2s larges, 1H chacun : -H de l'imidazole); 7,77 (d, J=8 Hz, 1H : -H6); 7,88 (d, J=1 Hz, 1H : -H9); 8,77 et 8,86 (2s, 1H chacun : -NH-CO-NH-); 12,35 (s large, 1H: -CO-NH-)].

### EXEMPLE 23

A une suspension de 1,5 g de monochlorhydrate de 5H,10H-8-amino-imidazo[1,2-a]indéno[1,2-e]pyrazine-4-one dans 50 ml de diméthylformamide sont additionnés 1,6 g de triéthylamine, puis 1 g d'anhydride acétique. Après 6 heures de reflux, le mélange est refroidi et versé dans 200 ml d'eau. Le précipité formé est filtré et cristallisé à chaud dans 20 ml de diméthylformamide. Les cristaux sont séparés par filtration et lavés deux fois avec 50 ml d'éther éthylique. On obtient ainsi 1,4 g de 5H,10H-8-acétamidoimidazo[1,2-a]indéno[1,2-e]pyrazine-4-one sous forme d'un solide beige rosé fondant au-dessus de 260°C [Spectre de R.M.N.: (300 MHz; DMSO d6; d en ppm) : 2,10 (s, 3H : -NH-CO-CH3); 4,01 (s, 2H : -CH2- en 10); 7,52 (dd, J=8 et 1 Hz, 1H : -H7); 7,57 et de 7,90 à 7,95 (respectivement s et mt, 1H chacun: -H de l'imidazole); 7,75 (d, J=8 Hz, 1H : -H6); de 7,90 à 7,95 (mt, 1H: -H9); 10,03 (s large, 1H : -NH-CO-CH3); 12,35 (s large, 1H : -CO-NH-)].

### EXEMPLE 24

A un mélange de 1,5 g de monochlorhydrate de 5H,10H-8-aminoimidazo[1,2-a]indéno[1,2-e]pyrazine-4-one et de 1,6 g de triéthylamine est ajouté goutte à goutte 1,3 g de chlorure de méthylsulfonyle. Le milieu réactionnel est maintenu 4 heures sous agitation à une température voisine de 20°C, puis il est filtré. Le solide résultant est lavé par 80 ml d'eau et par 30 ml de diméthylformamide chaud. Il est ensuite dissous dans 4 ml d'eau bouillante et il précipite par addition de 10 ml de diméthylformamide. Après filtration, on obtient 192 mg de monochlorhydrate de 5H,10H-8-diméthylaminométhylèneamino-imidazo[1,2-a]indéno[1,2-e]pyrazine-4-one sous forme de solide rosé fondant au-dessus de 260°C.[Spectre de R.M.N. : (250 MHz; DMSO d6; à une température de 343 K; δ en ppm) : 3,40 [mf, 6H : -N(CH3)2]; 4,10 (s, 2H : -CH2- en 10); 7,55 (dd, J=8 et 1,5 Hz, 1H : -H7); 7,58 et 7,94 (2d, J=1 Hz, 1H chacun : -H de l'imidazole); 7,76 (d, J=1,5 Hz, 1H : -H9); 7,92 (d, J=8 Hz, 1H : -H6); 8,75 (s, 1H : =N-CH-); 11,60 [mf, 1H : -N+H(CH3)2Cl-]; 12,22 (mf, 1H : -CO-NH-)].

### EXEMPLE 25

A une solution de 1,5 g de 1-[2-(6-fluoro-1-oxo-indanyl)]imidazole-2-carboxylate d'éthyle en solution dans 50 ml d'acide acétique, on ajoute progressivement 46,7 g d'acétate d'ammonium. Le milieu réactionnel est porté à reflux pendant 30 mn puis refroidi à une température voisine de 20°C. Le précipité formé est filtré, lavé à l'eau puis à l'éther isopropylique. On obtient ainsi 0,7 g de 7-fluoro-5H,10H-imidazo[1,2-a]indéno[1,2-e]pyrazine-4-one sous forme d'un solide beige fondant à une température supérieure à 260°C [RMN 1H (DMSO, 200 MHz), δ ppm : 4,00 (doublet large, 2H : 10-CH₂), 7,14 (triplet dédoublé, J = 8,5 Hz et 2,5 Hz, 1H : 8-ArH), 7,60 (doublet de doublet, J = 8,5 Hz et 5 Hz, 1H : 9-ArH et doublet large, J = 1 Hz, 1H : H-imidazole), 7,70 (doublet de doublet, J = 8,5 Hz et 2,5 Hz, 1H : 5-ArH), 8,00 (doublet, J = 1 Hz, 1H : H-imidazole), 12,35 (massif, 1H, NH)].

Le 1-[2-(6-fluoro-1-oxo-indanyl)]imidazole-2-carboxylate d'éthyle peut être préparé de la façon suivante : 12,8 g d'imidazole-2-carboxylate d'éthyle et 10,1 g 2-bromo-6-fluoroindanone sont chauffés à 130°C pendant 20 minutes. Après refroidissement à une température voisine de 20°C, le mélange est repris dans 100 ml de dichlorométhane, lavé 2 fois par 30 ml de soude 0,1N puis 3 fois par 40 ml d'eau distillée. Après séchage de la phase organique sur sulfate de magnésium, celle ci est traitée au noir décolorant et concentrée à sec sous pression réduite (15 mm Hg; 2 kPa). Le produit brut ainsi obtenu (10,6 g) est purifié par flash-chromatographie sur colonne de silice en utilisant un mélange de dichlorométhane et de méthanol (98/2 en volumes) comme éluant. On obtient 4,8 g de produit attendu sous forme de meringue beige (Rf = 0,19, .chromatographie sur couche mince de gel de silice, éluant : dichlorométhane-acétate d'éthyle (70-30 en volumes)).

La 2-bromo-6-fluoroindanone peut être préparée de la façon suivante : une solution de 6,4 g de brome dans 30 ml d'acide acétique est ajoutée goutte à goutte en 25 mn à 20°C à une solution de 12 g de 6-fluoroindanone et de 0,05 ml d'une solution aqueuse d'acide bromhydrique à 47% dans 100 ml d'acide acétique. Après 5 heures d'agitation, le mélange est versé sur de la glace pilée. Le précipité formé est filtré, lavé à l'eau distillée puis avec de l'éther de pétrole. On obtient ainsi 10,1 g de produit attendu sous forme de solide beige fondant à 98°C.

La 6-fluoroindanone peut être obtenue selon la méthode décrite par R. Seka et W. Kellermann, Chem. Ber. 75B, 1730 (1942).

### EXEMPLE 26

0,76 g de 1-[2-(1-oxo-indanyl)]imidazole-2-carboxamide et 3 ml d'un solution aqueuse d'acide chlorhydrique 10 N sont agités à une température voisine de 20°C pendant 10 minutes. Le milieu réactionnel hétérogène est dilué avec 27 ml d'eau et porté à reflux. On additionne ensuite au reflux 200 ml d'une solution aqueuse d'acide chlorhydrique 1 N jusqu'à dissolution totale de l'insoluble. Après refroidissement à une température voisine de 20°C et précipitation, on obtient le chlorhydrate du 5H,10H-imidazo[1,2-a] indéno[1,2-e] pyrazine-4-one se décomposant sans fondre à 350°C [Spectre infra-rouge (KBr), bandes caractéristiques en cm⁻¹ : 3550; 3250; 3200; 3140; 3125; 3060; 3020; 3000; 2400; 1705; 1645; 1555; 1505; 1460; 1385; 765 et 725].

Le 1-[2-(1-oxo-indanyl)]imidazole-2-carboxamide peut être préparé de la manière suivante : 1,35 g de 1-[2-(1-oxo-indanyl)]imidazole-2-carboxylate d'éthyle dissous dans 15 ml de méthanol sont introduits dans 25 ml d'une solution 2,5 N de méthanol ammoniacal et la solution est conservée pendant 20 heures à une température voisine de 20°C puis concentrée à sec sous pression réduite (15 mm de Hg; 2 kPa) à 40°C. Le produit obtenu est mis en suspension dans 30 ml d'oxyde d'isopropyle, filtré, lavé 2 fois par 20 ml au total d'oxyde d'isopropyle puis séché sous pression réduite (15 mm de Hg; 2 kPa) à une température voisine de 20°C. On obtient ainsi 1,2 g de 1-[2-(1-oxo-indanyl)]imidazole-2-carboxamide sous forme d'un solide fondant à 183°C.

Le 1-[2-(1-oxo-indanyl)]imidazole-2-carboxylate d'éthyle peut être préparé de la manière suivante : à une suspension de 0,4 g d'hydrure de sodium à 80 % dans 5 ml de diméthylformamide anhydre maintenue sous atmosphère d'azote, on ajoute, goutte à goutte, en 10 minutes, à une température comprise entre 20 et 25°C, une solution de 1,4 g d'imidazole-2-carboxylate d'éthyle dans 15 ml de diméthylformamide. Après 15 minutes d'agitation on ajoute, goutte à goutte, en 10 minutes, une solution de 3 g de 2-bromo-1-indanone dans 20 ml de diméthylformamide. Le mélange est agité pendant 1 heure puis, après addition lente de 10 ml d'eau, versé sur 400 ml d'eau distillée et extrait 3 fois par 300 ml au total de chloroforme. Les extraits organiques sont réunis, lavés avec 100 ml d'eau distillée, séchés sur sulfate de sodium anhydre et concentrés à sec sous pression réduite (15 mm de Hg, 2 kPa) à 35°C. Le produit obtenu (3,55 g) est chromatographié sur 215 g de gel de silice neutre (0,020-0,045 mm) contenus dans une colonne de 4,1 cm de diamètre en éluant sous pression avec un mélange dichlorométhane-acétate d'éthyle (60-40 en volumes) et en recueillant des fractions de 90 ml. Les fractions 16 à 25 sont réunies, concentrées à sec sous pression réduite (15 mm de Hg, 2 kPa) à 35°C. On obtient ainsi 1,89 g de 1-[2-(1-oxo-indanyl)]imidazole-2-carboxylate d'éthyle sous forme d'un solide fondant à 116°C.

Les médicaments selon l'invention sont constitués par un composé de formule (I) ou un sel d'un tel composé, à l'état pur ou sous forme d'une composition dans laquelle il est associé à tout autre produit pharmaceutiquement compatible, pouvant être inerte ou physiologiquement actif. Les médicaments selon l'invention peuvent être employés par voie orale, parentérale, rectale ou topique.

Comme compositions solides pour administration orale, peuvent être utilisés des comprimés, des pilules, des poudres (capsules de gélatine, cachets) ou des granulés. Dans ces compositions, le principe actif selon l'invention est mélangé à un ou plusieurs diluants inertes, tels que amidon, cellulose, saccharose, lactose ou silice, sous courant d'argon. Ces compositions peuvent également comprendre des substances autres que les diluants, par exemple un ou plusieurs lubrifiants tels que le stéarate de magnésium ou le talc, un colorant, un enrobage (dragées) ou un vernis.

Comme compositions liquides pour administration orale, on peut utiliser des solutions, des suspensions, des émulsions, des sirops et des élixirs pharmaceutiquement acceptables contenant des diluants inertes tels que l'eau, I'éthanol, le glycérol, les huiles végétales ou l'huile de paraffine. Ces compositions peuvent comprendre des substances autres que les diluants, par exemple des produits mouillants, édulcorants, épaississants, aromatisants ou stabilisants.

Les compositions stériles pour administration parentérale, peuvent être de préférence des solutions aqueuses ou non aqueuses, des suspensions ou des émulsions. Comme solvant ou véhicule, on peut employer l'eau, le propylèneglycol, un polyéthylèneglycol, des huiles végétales, en particulier l'huile d'olive, des esters organiques injectables, par exemple l'oléate d'éthyle ou d'autres solvants organiques convenables. Ces compositions peuvent également contenir des adjuvants, en particulier des agents mouillants, isotonisants, émulsifiants, dispersants et stabilisants. La stérilisation peut se faire de plusieurs façons, par exemple par filtration aseptisante, en incorporant à la composition des agents stérilisants, par irradiation ou par chauffage. Elles peuvent également être préparées sous forme de compositions solides stériles qui peuvent être dissoutes au moment de l'emploi dans de l'eau stérile ou tout autre milieu stérile injectable.

Les compositions pour administration rectale sont les suppositoires ou les capsules rectales qui contiennent, outre le produit actif, des excipients tels que le beurre de cacao, des glycérides semi-synthétiques ou des polyéthylèneglycols.

Les compositions pour administration topique peuvent être par exemple des crèmes, lotions, collyres, collutoires, gouttes nasales ou aérosols.

En thérapeutique humaine, les composés selon l'invention sont particulièrement utiles pour le traitement et/ou la prévention des conditions qui requièrent l'administration d'un antagoniste du récepteur AMPA ou d'un antagoniste du récepteur NMDA. Ces composés sont notamment utiles pour traiter ou prévenir toutes les ischémies et en particulier l'ischémie cérébrale, les effets dus à une anoxie, l'évolution de maladies neurodégénératives, de la chorée d'HUNTINGTON, de la maladie d'ALZHEIMER, de la sclérose latérale amyotrophique, de l'atrophie olivo-pontocérébelleuse et de la maladie de PARKINSON, vis-à-vis des manifestations épileptogènes et/ou convulsives, pour le traitement des traumatismes cérébraux et spinaux, de l'anxiété, de la dépression, de la schizophrénie, en tant qu'analgésiques, antianorexiques, antimigraineux, antiémétiques et pour traiter les empoisonnements par des neurotoxines ou d'autres substances agonistes du récepteur NMDA, ainsi que les troubles neurologiques associés aux maladies virales telles que le sida, la rage, la rougeole et le tétanos. Ces composés sont aussi utiles pour la prévention des symptomes d'abstinence aux drogues et à l'alcool et de l'inhibition de l'accoutumance et de la dépendance aux opiacés.

Les doses dépendent de l'effet recherché, de la durée du traitement et de la voie d'administration utilisée; elles sont généralement comprises entre 10 mg et 100 mg par jour par voie orale pour un adulte avec des doses unitaires allant de 5 mg à 50 mg de substance active.

D'une façon générale, le médecin déterminera la posologie appropriée en fonction de l'âge, du poids et de tous les autres facteurs propres au sujet à traiter.
Les exemples suivants illustrent des compositions selon l'invention :

### EXEMPLE A

On prépare, selon la technique habituelle, des gélules dosées à 50 mg de produit actif ayant la composition suivante :
- Composé de formule (I) 50 mg
- Cellulose 18 mg
- Lactose 55 mg
- Silice colloïdale 1 mg
- Carboxyméthylamidon sodique 10 mg
- Talc 10 mg
- Stéarate de magnésium 1 mg

### EXEMPLE B

On prépare selon la technique habituelle de comprimés dosés à 50 mg de produit actif ayant la composition suivante :
- Composé de formule (I) 50 mg
- Lactose 104 mg
- Cellulose 40 mg
- Polyvidone 10 mg
- Carboxyméthylamidon sodique 22 mg
- Talc 10 mg
- Stéarate de magnésium 2 mg
- Silice colloïdale 2 mg
- Mélange d'hydroxyméthylcellulose, glycérine, oxyde de titane (72-3,5-24,5) q.s.p. 1 comprimé pelliculé terminé à 245 mg

### EXEMPLE C

On prépare une solution injectable contenant 10 mg de produit actif ayant la composition suivante :
- Composé de formule (I) 10 mg
- Acide benzoïque 80 mg
- Alcool benzylique 0,06 ml
- Benzoate de sodium 80 mg
- Ethanol à 95 % 0,4 ml
- Hydroxyde de sodium 24 mg
- Propylène glycol 1,6 ml
- Eau q.s.q. 4 ml

## Revendications

1. Composés de formule : 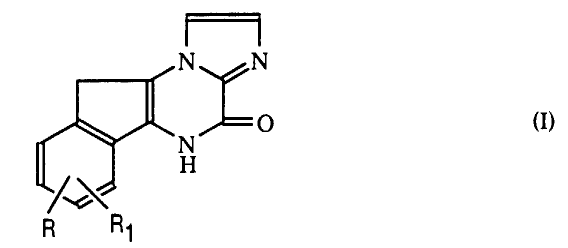 dans laquelle R et R₁, identiques ou différents, représentent un atome d'hydrogène ou d'halogène ou un radical alkyle, alcoxy, amino, acylamino, phényluréido, -N=CH-N(R₂)R₃, nitro, imidazolyle, phényle, SO₃H ou cyano, R₂ et R₃, identiques ou différents, représentent chacun un radical alkyle, étant entendu que les radicaux alkyle et alcoxy contiennent 1 à 4 atomes de carbone en chaîne droite ou ramifiée et les portions acyle contiennent 2 à 5 atomes de carbone, leurs isomères purs et les mélanges de ces isomères lorsque R et/ou R₁ représente un radical -N=CH-N(R₂)R₃ et leurs sels.

2. Composés de formule (I) selon la revendication 1 pour lesquels soit R représente un atome d'hydrogène et R₁ est en position -7, -8 ou -9 et représente un atome d'hydrogène ou d'halogène ou un radical alkyle, alcoxy, amino, acylamino, phényluréido, -N=CH-N(R₂)R₃, nitro, imidazolyle, phényle ou SO₃H; soit R représente un atome d'halogène et R₁ représente un atome d'halogène ou un radical nitro, étant entendu que les radicaux alkyle et alcoxy contiennent 1 à 4 atomes de carbone en chaîne droite ou ramifiée et les portions acyle contiennent 2 à 5 atomes de carbone, leurs isomères purs et les mélanges de ces isomères lorsque R₁ représente un radical -N=CH-N(R₂)R₃ et leurs sels.

3. Procédé de préparation des composés de formule (I) selon la revendication 1 caractérisé en ce que l'on désalkyle et désalifie un dérivé de formule : 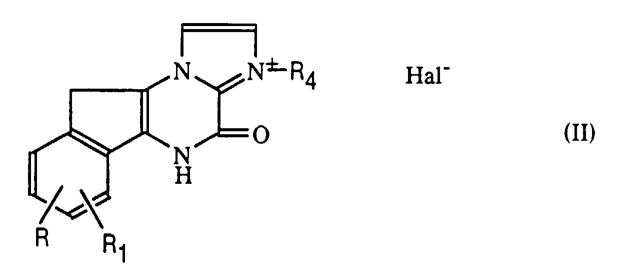 dans laquelle R et R₁ ont les mêmes significations que dans la revendication 1, R₄ représente un radical alkyle et Hal représente un atome d'halogène, isole le produit et le transforme éventuellement en sel.

4. Procédé de préparation des composés de formule (I) selon la revendication 1 caractérisé en ce que l'on cyclise un dérivé de formule : 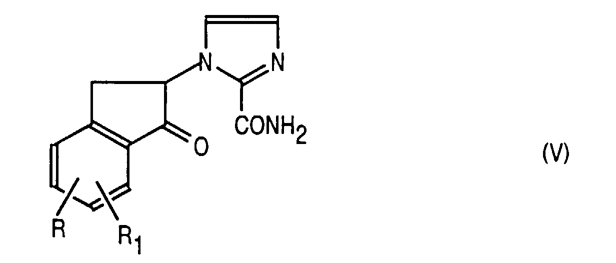 dans laquelle R et R₁ ont les mêmes significations que dans la revendication 1, isole le produit et le transforme éventuellement en sel.

5. Procédé de préparation des composés de formule (I) selon la revendication 1 pour lesquels R et/ou R₁ représentent un radical nitro caractérisé en ce que l'on nitre un composé de formule (I) correspondant pour lequel R et/ou R₁ représentent un atome d'hydrogène, isole le produit et le transforme éventuellement en sel.

6. Procédé de préparation des composés de formule (I) selon la revendication 1 pour lesquels R et/ou R₁ représentent un radical amino caractérisé en ce que l'on réduit un composé de formule (I) correspondant pour lequel R et/ou R₁ représentent un radical nitro, isole le produit et le transforme éventuellement en sel.

7. Procédé de préparation des composés de formule (I) selon la revendication 1 pour lesquels R et/ou R₁ représentent un radical SO₃H caractérisé en ce que l'on sulfone un dérivé de formule (I) correspondant pour lequel R et/ou R₁ représentent un atome d'hydrogène, isole le produit et le transforme éventuellement en sel.

8. Procédé de préparation des composés de formule (I) selon la revendication 1 pour lesquels R et/ou R₁ représentent un radical acylamino caractérisé en ce que l'on acyle un composé de formule (I) correspondant pour lequel R et/ou R₁ représentent un radical amino, isole le produit et le transforme éventuellement en sel.

9. Procédé de préparation des composés de formule (I) selon la revendication 1 pour lesquels R et/ou R₁ représentent un radical phényluréido caractérisé en ce que l'on fait réagir l'isocyanate de phényle sur un composé de formule (I) correspondant pour lequel R et/ou R₁ représentent un radical amino, isole le produit et le transforme éventuellement en sel.

10. Procédé de préparation des composés de formule (I) selon la revendication 1 pour lesquels R et/ou R₁ représentent un radical -N=CH-N(R₂)R₃ caractérisé en ce que l'on fait réagir un composé de formule (I) correspondant pour lequel R et/ou R₁ représentent un radical amino sur un amide HCO-N(R₂)R₃ dans lequel R₂ et R₃ ont les mêmes significations que dans la revendication 1, isole le produit et le transforme éventuellement en sel.

11. Médicaments comprenant en tant que principe actif au moins un composé de formule (I) selon la revendication 1 ou un sel d'un tel composé.

12. Médicaments comprenant en tant que principe actif au moins un composé selon la revendication 2 ou un sel d'un tel composé.

13. Médicaments selon les revendications 11 et 12 utiles comme antagonistes du récepteur AMPA.

14. Médicaments selon les revendications 11 et 12 utiles comme antagonistes du récepteur NMDA.

15. Composés de formule : 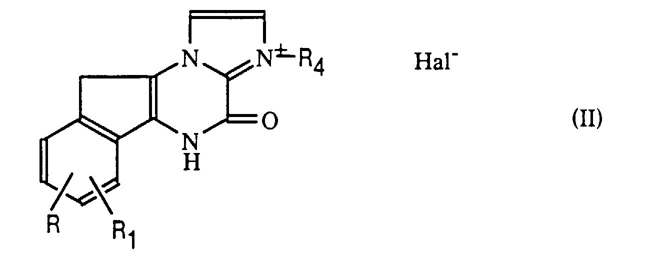 dans laquelle R et R₁, identiques ou différents, représentent un atome d'hydrogène ou d'halogène ou un radical alkyle, alcoxy, amino, acylamino, phényluréido, -N=CH-N(R₂)R₃, nitro, imidazolyle, phényle, SO₃H ou cyano, R₂ et R₃, identiques ou différents, représentent chacun un radical alkyle, R₄ représente un radical alkyle et Hal représente un atome d'halogène, étant entendu que les radicaux alkyle et alcoxy contiennent 1 à 4 atomes de carbone en chaîne droite ou ramifiée et les radicaux acyle contiennent 2 à 5 atomes de carbone.

## Patentansprüche

1. Verbindungen der Formel (I) 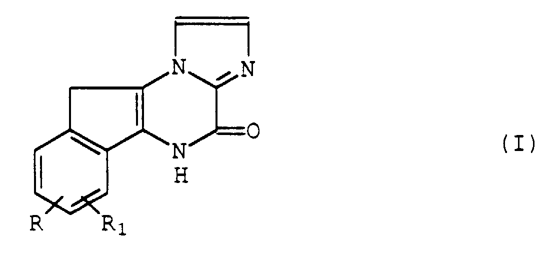 in der
R und R₁, gleich oder verschieden, ein Wasserstoffatom oder Halogenatom oder einen Rest Alkyl, Alkoxy, Amino, Acylamino, Phenylureido, -N=CH-N(R₂)R₃, Nitro, Imidazolyl, Phenyl, SO₃H oder Cyano darstellen, R₂ und R₃, gleich oder verschieden, jeweils einen Rest Alkyl bedeuten, mit der Maßgabe, daß die Reste Alkyl und Alkoxy 1 bis 4 Kohlenstoffatome in gerader oder verzweigter Kette enthalten und die Teile Acyl 2 bis 5 Kohlenstoffatome enthalten, sowie ihre reinen Isomeren und die Mischungen dieser Isomeren, wenn R und/oder R₁ einen Rest -N=CH-N(R₂)R₃ darstellt, und ihre Salze.

2. Verbindungen der Formel (I) nach Anspruch 1, worin entweder R ein Wasserstoffatom darstellt und R₁ in Position -7, -8 oder -9 ist und ein Wasserstoffatom oder Halogenatom oder einen Rest Alkyl, Alkoxy, Amino, Acylamino, Phenylureido, -N=CH-N(R₂)R₃, Nitro, Imidazolyl, Phenyl oder SO₃H bedeutet,
oder R ein Halogenatom darstellt und R₁ ein Halogenatom oder einen Rest Nitro bedeutet, mit der Maßgabe, daß die Reste Alkyl und Alkoxy 1 bis 4 Kohlenstoffatome in gerader oder verzweigter Kette enthalten und die Teile Acyl 2 bis 5 Kohlenstoffatome enthalten, sowie ihre reinen Isomeren und die Mischungen dieser Isomeren, wenn R₁ einen Rest -N=CH-N(R₂)R₃ darstellt, und ihre Salze.

3. Verfahren zur Herstellung der Verbindungen der Formel (I) nach Anspruch 1, dadurch gekennzeichnet, daß man ein Derivat der Formel (II) 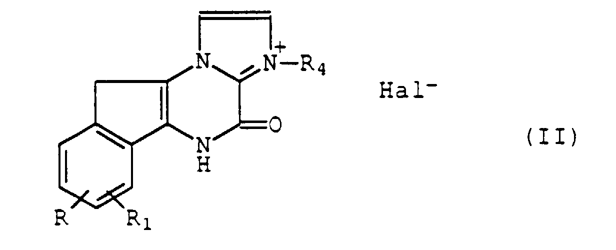 in der R und R₁ die gleichen Bedeutungen wie in Anspruch 1 besitzen, R₄ einen Rest Alkyl darstellt und Hal ein Halogenatom bedeutet, dealkyliert und aus seinem Salz freisetzt, das Produkt isoliert und gegebenenfalls in Salz überführt.

4. Verfahren zur Herstellung der Verbindungen der Formel (I) nach Anspruch 1, dadurch gekennzeichnet, daß man ein Derivat der Formel (V) 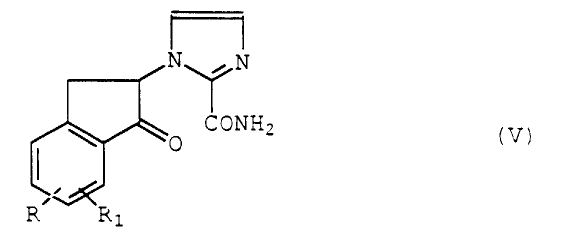 in der R und R₁ die gleichen Bedeutungen wie in Anspruch 1 besitzen, cyclisiert, das Produkt isoliert und gegebenenfalls in Salz überführt.

5. Verfahren zur Herstellung der Verbindungen der Formel (I) nach Anspruch 1, worin R und/oder R₁ einen Rest Nitro darstellen, dadurch gekennzeichnet, daß man eine entsprechende Verbindung der Formel (I), in der R und/oder R₁ ein Wasserstoffatom bedeuten, nitriert, das Produkt isoliert und gegebenenfalls in Salz überführt.

6. Verfahren zur Herstellung der Verbindungen der Formel (I) nach Anspruch 1, worin R und/oder R₁ einen Rest Amino darstellen, dadurch gekennzeichnet, daß man eine entsprechende Verbindung der Formel (I), in der R und/oder R₁ einen Rest Nitro bedeuten, reduziert, das Produkt isoliert und gegebenenfalls in Salz überführt.

7. Verfahren zur Herstellung der Verbindungen der Formel (I) nach Anspruch 1, worin R und/oder R₁ einen Rest SO₃H darstellen, dadurch gekennzeichnet, daß man eine entsprechende Verbindung der Formel (I), in der R und/oder R₁ ein Wasserstoffatom bedeuten, sulfoniert, das Produkt isoliert und gegebenenfalls in Salz überführt.

8. Verfahren zur Herstellung der Verbindungen der Formel (I) nach Anspruch 1, worin R und/oder R₁ einen Rest Acylamino darstellen, dadurch gekennzeichnet, daß man eine entsprechende Verbindung der Formel (I), in der R und/oder R₁ einen Rest Amino bedeuten, acyliert, das Produkt isoliert und gegebenenfalls in Salz überführt.

9. Verfahren zur Herstellung der Verbindungen der Formel (I) nach Anspruch 1, worin R und/oder R₁ einen Rest Phenylureido darstellen, dadurch gekennzeichnet, daß man Phenylisocyanat mit einer entsprechenden Verbindung der Formel (I), in der R und/oder R₁ einen Rest Amino bedeuten, zur Reaktion bringt, das Produkt isoliert und gegebenenfalls in Salz überführt.

10. Verfahren zur Herstellung der Verbindungen der Formel (I) nach Anspruch 1, worin R und/oder R₁ einen Rest -N=CH-N(R₂)R₃ darstellen, dadurch gekennzeichnet, daß man eine entsprechende Verbindung der Formel (I), in der R und/oder R₁ einen Rest Amino bedeuten, mit einem Amid HCO-N(R₂)R₃, worin R₂ und R₃ die gleichen Bedeutungen wie in Anspruch 1 besitzen, zur Reaktion bringt, das Produkt isoliert und gegebenenfalls in Salz überführt.

11. Arzneimittel, umfassend als Wirkstoff mindestens eine Verbindung der Formel (I) nach Anspruch 1 oder ein Salz einer derartigen Verbindung.

12. Arzneimittel, umfassend als Wirkstoff mindestens eine Verbindung nach Anspruch 2 oder ein Salz einer derartigen Verbindung.

13. Arzneimittel nach Anspruch 11 und 12, geeignet als Antagonisten des Rezeptors AMPA.

14. Arzneimittel nach Anspruch 11 und 12, geeignet als Antagonisten des Rezeptors NMDA.

15. Verbindungen der Formel (II) 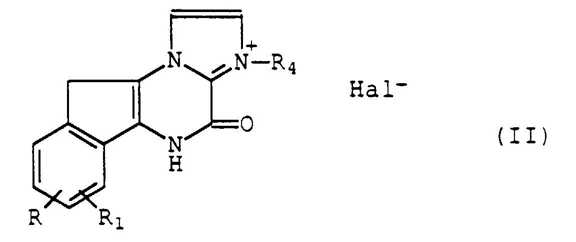 in der
R und R₁, gleich oder verschieden, ein Wasserstoffatom oder Halogenatom oder einen Rest Alkyl, Alkoxy, Amino, Acylamino, Phenylureido, -N=CH-N(R₂)R₃, Nitro, Imidazolyl, Phenyl, SO₃H oder Cyano darstellen, R₂ und R₃, gleich oder verschieden, jeweils einen Rest Alkyl bedeuten, R₄ ein Rest Alkyl ist und Hal ein Halogenatom darstellt, mit der Maßgabe, daß die Reste Alkyl und Alkoxy 1 bis 4 Kohlenstoffatome in gerader oder verzweigter Kette enthalten und die Teile Acyl 2 bis 5 Kohlenstoffatome enthalten.

## Claims

1. Compounds of formula: 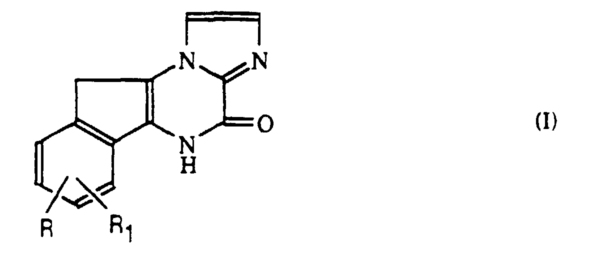 in which R and R₁, which are identical or different, represent a hydrogen or halogen atom or an alkyl, alkoxy, amino, acylamino, phenylureido, -N=CH-N(R₂)R₃, nitro, imidazolyl, phenyl, SO₃H or cyano radical, R₂ and R₃, which are identical or different, each represent an alkyl radical, it being understood that the alkyl and alkoxy radicals contain 1 to 4 straight- or branched-chain carbon atoms and the acyl portions contain 2 to 5 carbon atoms, their pure isomers and the mixtures of these isomers when R and/or R₁ represent an -N=CH-N(R₂)R₃ radical, and their salts.

2. Compounds of formula (I) according to claim 1, in which either R represents a hydrogen atom and R₁ is at the 7-, 8- or 9-position and represents a hydrogen or halogen atom or an alkyl, alkoxy, amino, acylamino, phenylureido, -N=CH-N(R₂)R₃, nitro, imidazolyl, phenyl or SO₃H radical; or R represents a halogen atom and R₁ represents a halogen atom or a nitro radical, it being understood that the alkyl and alkoxy radicals contain 1 to 4 straight- or branched-chain carbon atoms and the acyl portions contain 2 to 5 carbon atoms, their pure isomers and the mixtures of these isomers when R₁ represents an -N=CH-N(R₂)R₃ radical, and their salts.

3. Process for the preparation of the compounds of formula (I) according to claim 1, characterized in that a derivative of formula: 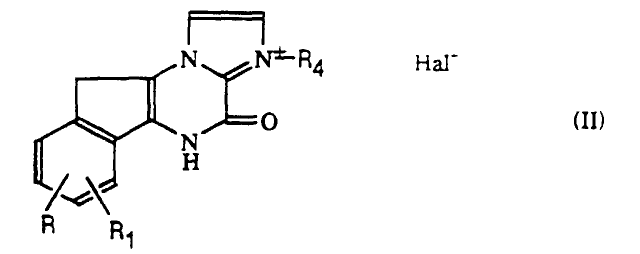 in which R and R₁ have the same meanings as in claim 1, R₄ represents an alkyl radical and Hal represents a halogen atom, is dealkylated and desalified, the product is isolated and is optionally converted into a salt.

4. Process for the preparation of the compounds of formula (I) according to claim 1, characterized in that a derivative of formula: 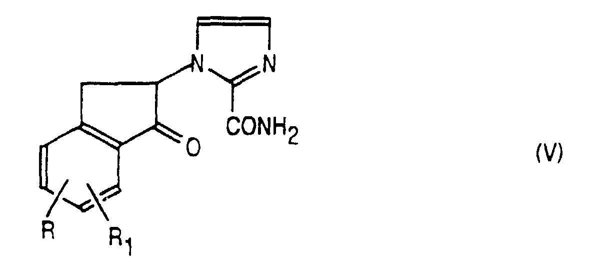 in which R and R₁ have the same meanings as in claim 1, is cyclized, the product is isolated and is optionally converted into a salt.

5. Process for the preparation of the compounds of formula (I) according to claim 1 in which R and/or R₁ represent a nitro radical, characterized in that a corresponding compound of formula (I), in which R and/or R₁ represent a hydrogen atom, is nitrated, the product is isolated and is optionally converted into a salt.

6. Process for the preparation of the compounds of formula (I) according to claim 1 in which R and/or R₁ represent an amino radical, characterized in that a corresponding compound of formula (I), in which R and/or R₁ represent a nitro radical, is reduced, the product is isolated and is optionally converted into a salt.

7. Process for the preparation of the compounds of formula (I) according to claim 1 in which R and/or R₁ represent an SO₃H radical, characterized in that a corresponding derivative of formula (I), in which R and/or R₁ represent a hydrogen atom, is sulphonated, the product is isolated and is optionally converted into a salt.

8. Process for the preparation of the compounds of formula (I) according to claim 1 in which R and/or R₁ represent an acylamino radical, characterized in that a corresponding compound of formula (I), in which R and/or R₁ represent an amino radical, is acylated, the product is isolated and is optionally converted into a salt.

9. Process for the preparation of the compounds of formula (I) according to claim 1 in which R and/or R₁ represent a phenylureido radical, characterized in that phenyl isocyanate is reacted with a corresponding compound of formula (I), in which R and/or R₁ represent an amino radical, the product is isolated and is optionally converted into a salt.

10. Process for the preparation of the compounds of formula (I) according to claim 1 in which R and/or R₁ represent an -N=CH-N(R₂)R₃ radical, characterized in that a corresponding compound of formula (I), in which R and/or R₁ represent an amino radical, is reacted with an amide HCO-N(R₂)R₃, in which R₂ and R₃ have the same meanings as in claim 1, the product is isolated and is optionally converted into a salt.

11. Medicaments comprising, as active principle, at least one compound of formula (I) according to claim 1, or a salt of such a compound.

12. Medicaments comprising, as active principle, at least one compound according to claim 2, or a salt of such a compound.

13. Medicaments according to claims 11 and 12, which are useful as antagonists of the AMPA receptor.

14. Medicaments according to claims 11 and 12, which are useful as antagonists of the NMDA receptor.

15. Compounds of formula: 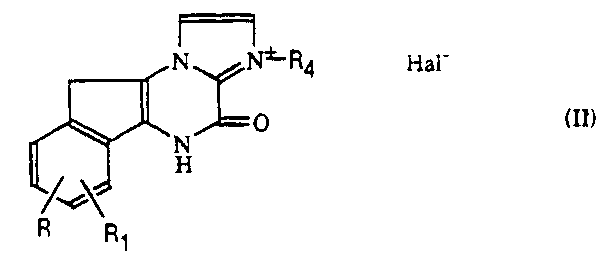 in which R and R₁, which are identical or different, represent a hydrogen or halogen atom or an alkyl, alkoxy, amino, acylamino, phenylureido, -N=CH-N(R₂)R₃, nitro, imidazolyl, phenyl, SO₃H or cyano radical, R₂ and R₃, which are identical or different, each represent an alkyl radical, R₄ represents an alkyl radical and Hal represents a halogen atom, it being understood that the alkyl and alkoxy radicals contain 1 to 4 straight- or branched-chain carbon atoms and the acyl radicals contain 2 to 5 carbon atoms.
